(19) Europäisches Patentamt
European Patent Office
Office européen des brevets

(11) Publication number: **0 009 800**
**B1**

(12) **EUROPEAN PATENT SPECIFICATION**

(45) Date of publication of patent specification: **23.02.83**

(21) Application number: **79103737.7**

(22) Date of filing: **02.10.79**

(51) Int. Cl.³: **C 07 D 243/04,**
A 61 K 31/55,
C 07 C 87/48,
C 07 C 93/14, C 07 C 91/42

(54) **4-Phenyl-1,3-benzodiazepines, method for their preparation, pharmaceutical compositions containing them, and the compounds for use as medicame: :s.**

(30) Priority: **05.10.78 US 948896**

(43) Date of publication of application:
**16.04.80 Bulletin 80/8**

(45) Publication of the grant of the patent:
**23.02.83 Bulletin 83/8**

(84) Designated Contracting States:
**AT BE CH DE FR GB IT NL SE**

(56) References cited:
**US - A - 3 681 340**

(73) Proprietor: **HOECHST AKTIENGESELLSCHAFT**
**Postfach 80 03 20**
**D-6230 Frankfurt/Main 80 (DE)**

(72) Inventor: **Martin, Lawrence Leo**
**No. 3, Box 81 Concord Road**
**Lebanon, N.J. (US)**
Inventor: **Worm, Manfred, Dr.**
**27 Erbsenacker**
**D-6200 Wiesbaden (DE)**
Inventor: **Crichlow, Charles Anthony**
**892 Davidson Road**
**Piscataway, New Jersey 08854 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 009 800 B1

# 4-Phenyl-1,3-benzodiazepines, a method for their preparation, pharmaceutical compositions containing them, and the compounds for use as medicaments

This invention relates to novel 4-phenyl-1,3-benzodiazepines, which are useful as antidepressants, analgetics, anticonvulsants, to methods of their preparation, and to pharmaceutical compositions containing such a compound as an active ingredient and to the compounds for use as medicaments.

The compounds of the present invention have not been described. Rodriguez et al., in U.S. Patent 3,681,340 entitled "1,3-Benzodiazepines" teaches that compounds of the formula

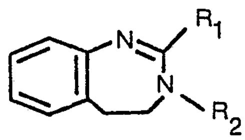

in which $R_1$ is hydrogen, free or etherified OH or SH, amino or an aliphatic, araliphatic or aromatic radical; $R_2$ is hydrogen or an acyl, aliphatic, araliphatic or aromatic radical; N-oxides and quaternaries and salts thereof, exhibit central nervous system depressing and coronary dilating effects.

The compounds of this invention have the formula I

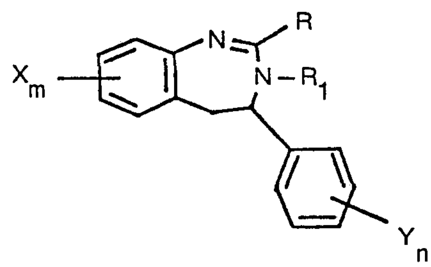

I

in which R and $R_1$ are the same or different and each can be hydrogen, alkyl of from 1 to 3 carbon atoms, cycloalkyl-$C_1$—$C_3$-alkyl wherein the cycloalkyl radical has 3—6 carbon atoms, particularly cyclohexylmethyl, or aralkyl having up to 8 carbon atoms, particularly benzyl. X and Y are the same or different and each can be chlorine, bromine, fluorine, alkoxy or alkyl each of from 1 to 3 carbon atoms, hydroxy, or trifluoromethyl; m is the integer 0, 1 or 2; and n is the integer 0, 1, 2 or 3. Also included within the scope of the present invention are the optical antipodes and the physiologically acceptable salts of the above-depicted compounds.

Novel intermediate compounds, utilized in the preparation of the benzodiazepines of this invention, have the formula II

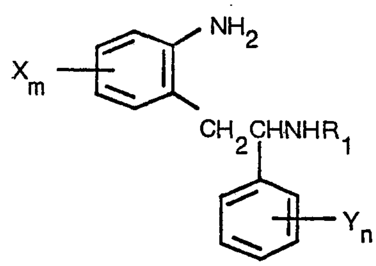

II

in which $R_1$, X, Y, m and n are as previously defined.

The physiologically acceptable salts of the present invention are acid addition salts which may be prepared from inorganic acids such as hydrochloric, hydrobromic, sulfuric, nitric, phosphoric and perchloric acids, as well as from organic acids such as tartaric, citric, acetic, succinic, maleic, furmaric and oxalic.

The compounds of the present invention can be prepared according to the following sequence of reactions in which R, $R_1$, X, Y, m and n are as previously defined, unless otherwise indicated.

a) A 2-(2-nitrophenyl)acetophenone of the formula III

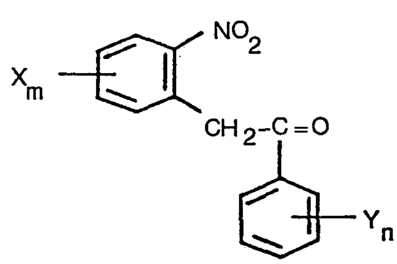

III

is converted to an oxime of the formula IV

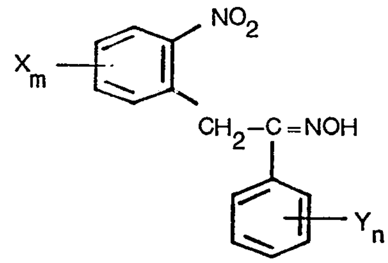

IV

by any convenient method known to the art. A preferred method involves refluxing the acetophenone in a mixture of ethyl alcohol, aqueous sodium acetate and hydroxylamine.

b) A compound of formula IV is acylated with acetic anhydride to provide the corresponding oxime acetate of the formula

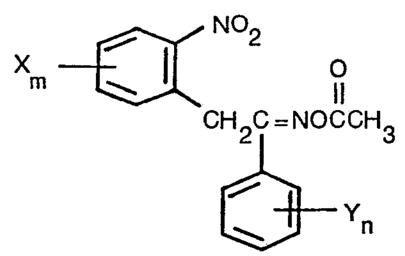

V

c) A compound of formula V is carefully reduced to provide the corresponding 2-nitro-a-phenylphenethylamine of the formula VI

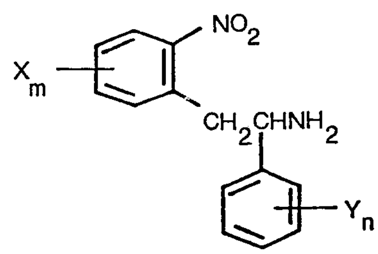

VI

The reducing agent used in this step must be compatible with the phenyl nitro group. Diborane is preferred. Additionally, this reduction is carried out in the presence of a solvent such as tetrahydrofuran and at a low temperature of from about 0°C to about 30°C or at ambient temperature.

d) A compound of formula VI is further reduced to provide the corresponding 2-amino-$\alpha$-phenylphenethylamine, a novel intermediate compound of the formula IIa

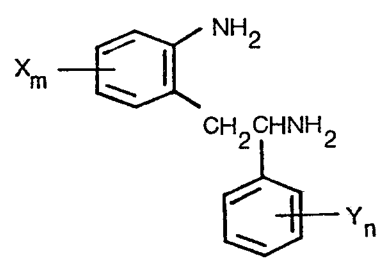

IIa

A preferred reducing method involves hydrogenation with a palladium on carbon catalyst or Adam's catalyst. Also other chemical reducing methods are suitable such as the use of tin and hydrochloric acid.

e) A compound of formula VI is converted by any method known to the art, to the corresponding amide of the formula

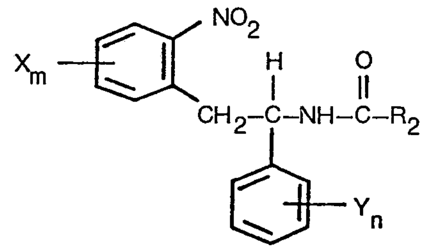

VII

wherein $R_2$ is hydrogen, $CH_3$ or $C_2H_5$. This conversion is carried out with an appropriate carboxylic acid anhydride or halide in the presence of a suitable solvent. Additionally, when $R_2$ is hydrogen, a preferred method is the mixed anhydride procedure utilizing a mixture of acetic anhydride and formic acid at a temperature of from about 0°C to 100°C with a suitable solvent such as benzene.

f) A compound of formula VII is reduced in a manner consistent with the procedure of step c, above, to provide the corresponding N-alkyl-α-phenyl-2-nitrophenylethylamine of the formula

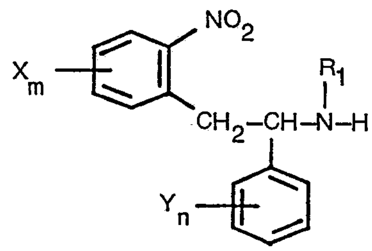

VIII

in which $R_1$ is alkyl.

g) A compound of formula VIII is reduced in a manner consistent with the procedure of step d, above, to provide the corresponding N-alkyl-2-amino-α-phenylphenethylamine, a novel intermediate of the present invention, of the formula

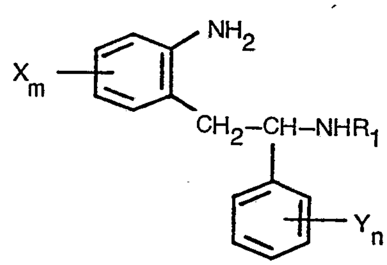

II

h) A compound of formula II or IIa is cyclized with a compound of the formula

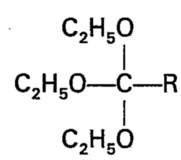

to provide the corresponding 1,3-benzodiazepine, a compound of the present invention, of the formula I

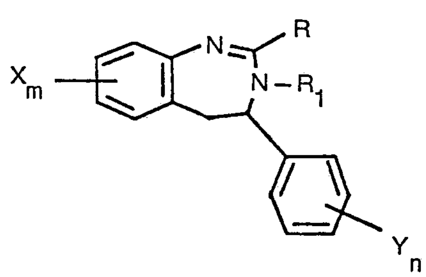

I

This cyclization is carried out in the presence of an acid catalyst such as ethanolic hydrochloric acid at a temperature of from 25°C. to reflux of the reaction mixture.

In each, of the above reaction steps, optimum conditions depend upon starting materials, solvents, catalysts and other reaction components, as will become more apparent in the examples given below.

Optical antipodes can be prepared by resolution with common resolving agents such as optically pure tartaric and sulfonic acids or synthesized from optically pure precursors.

Compounds depicted in formula I are either generally available or prepared as described below. O. Hromatka et al., Monatsh, *100*, (1969) describe the following synthesis in which X is chlorine or bromine:

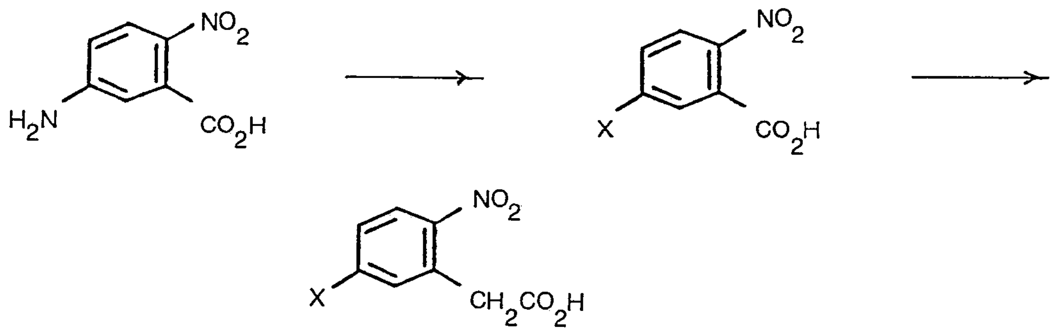

Routine manipulation of this synthesis would provide corresponding compounds in which X is fluorine, hydroxy, methoxy or cyano.

Additionally, 2,4-dinitroaniline can be treated by routine methods to ultimately provide a compound of the formula

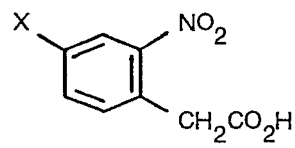

through the following precursors:

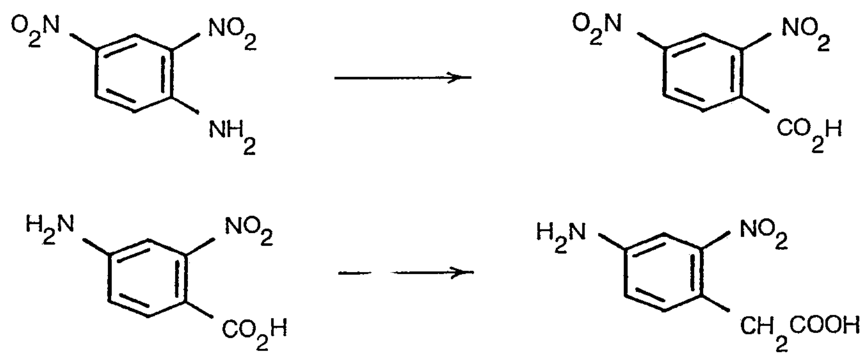

Thereafter, compounds of formula III can be prepared by Friedel-Crafts acylation as follows:

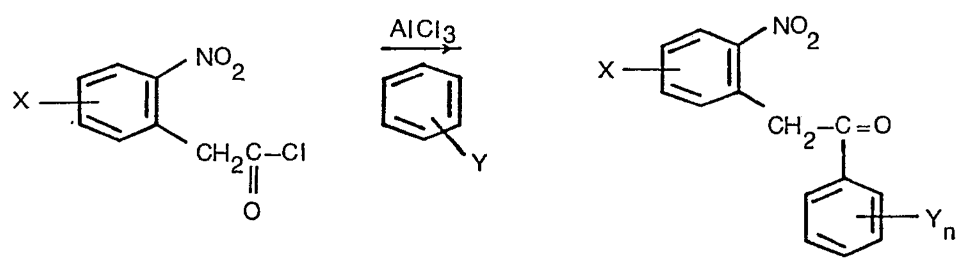


The utility of the compounds of the present invention in the treatment of depression in mammals is demonstrated by their ability to inhibit tetrabenazine induced depression in mice [International Journal of Neuropharmacology, *8*, 73 (1969)], a standard assay for useful anti-depressant properties. Thus, for instance, an intraperitoneal dose of 1.5 mg/kg of body weight and an oral dose of 2.1 mg/kg of body weight of 4,5-dihydro-2,3-dimethyl-4-phenyl-3H-1,3-benzodiazepine hydrochloride each demonstrate a 50% inhibition of ptosis of tetrabenazine-induced depression in mice. Also, an oral dose of 9.7 mg/kg of body weight of 4,5-dihydro-3-methyl-4-phenyl-3H-1,3-benzodiazepine hydrochloride and an intraperitoneal dose of 20 mg/kg of body weight of 4,5-dihydro-4-phenyl-3H-1,3-benzodiazepine hydrochloride demonstrate a similar inhibition in this assay. Finally, an intraperitoneal dose of 20 mg/kg body weight of 4,5-dihydro-2-methyl-4-phenyl-3H-1,3-benzodiazepine hydrochloride demonstrates a 30% inhibition in this assay. These data indicate that the compounds of the present invention would be useful as antidepressants in mammals when administered in amounts ranging from 0.01 to 100 mg/kg of body weight per day.

Compounds of the present invention are useful as analgetics due to their ability to alleviate pain in mammals, as demonstrated in the phenyl-2-quinone writhing assay in mice, a standard assay for analgesia [Proc. Soc. Exptl. Biol. Med., *95*, 729 (1957)]. Thus, for example, a subcutaneous dose of 21.3 mg/kg of body weight of 4,5-dihydro-4-phenyl-3H-1,3-benzodiazepine hydrochloride demonstrates a 50% inhibition of writhing produced in this assay. This datum illustrates that compounds of this invention are useful for alleviating pain in mammals when administered in amounts ranging from about 0.01 to about 100 mg/kg of body weight per day.

Compounds of the present invention are further useful as anticonvulsant agents for mammals, as determined by Woodbury, L. A. and Davenport, V. D. [Arch. Int. Pharmacodynam, *92*, pp 97—107 (1952)]. For example, 4,5-dihydro-3-methyl-4-phenyl-3H-1,3-benzodiazepine hydrochloride and 4,5-dihydro-4-phenyl-3H-1,3-benzodiazepine hydrochloride at an intraperitoneal dose of 13.4 and 19.4 mg/kg of body weight, respectively, each produce a 50% protection from the effect of supramaximal electroshock. These data illustrate that compounds of the present invention are useful in treating convulsions in mammals when administered in amounts ranging from about 0.01 to about 100 mg/kg of body weight per day.

Compounds of this invention include additionally to the Examples:

7-chloro-4,5-dihydro-2,3-dimethyl-4-phenyl-3H-1,3-benzodiazepine;
2,3-diethyl-7,8-difluoro-4,5-dihydro-4-phenyl-3H-1,3-benzodiazepine;
4,5-dihydro-2,3-dimethyl-4-(3-trifluoromethylphenyl)-3H-1,3-benzodiazepine;
4,5-dihydro-2-ethyl-4-phenyl-3H-1,3-benzodiazepine;
4,5-dihydro-4-phenyl-7-trifluoromethyl-3H-1,3-benzodiazepine;
4,5-dihydro-2,3-dimethyl-6-methoxy-4-(2-methoxy-phenyl)-3H-1,3-benzodiazepine;
4,5-dihydro-2-isopropyl-4-phenyl-3-n-propyl-3H-1,3-benzodiazepine;
7,8-dibromo-4,5-dihydro-2,3-dimethyl-4-(2-tolyl)-3H-1,3-benzodiazepine;
4,5-dihydro-9-hydroxy-4-(3-n-propylphenyl)-3H-1,3-benzodiazepine; and
7,8-dibromo-4-(2,3-difluorophenyl)-4,5-dihydro-2,3-dimethyl-3H-1,3-benzodiazepine.

Effective quantities of the compounds of the invention may be administered to a patient by any one of various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable addition salts for purposes of stability, convenience of crystallization, increased solubility and the like.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compound, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0—300 milligrams of active compound.

The tablets, pills, capsules, troches and the like may also contain the following ingredients: a binder such as microcrystalline cellulose, gum tragacanth or gelatin; an excipient such as starch or lactose, a disintegrating agent such as alginic acid, Primogel, corn starch and the like; a lubricant such as magnesium stearate or Sterotex; a glidant such as colloidal silicon dioxide; and a sweetening agent such as sucrose or saccharin may be added or a flavoring agent such as peppermint, methyl salicylat, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus, tablets or pills may be coated with sugar, shellac, or other enteric coating agents. A syrup may contain, in addition to the active compounds, sucrose as a sweetening agent, and certain preservatives, dyes and colorings and flavors. Materials used in preparing these various compositions should be pharmaceutically pure and non-toxic in the amounts used.

For the purpose of parenteral therapeutic adminstration, the active compounds of the invention may be incorporated into a solution or suspension. These preparations should contain at least 0.1% of active compound, but may be varied to be between 0.5 and about 5% of the weight thereof. The amount of active compounds in such compositions is such that a suitable dosage will be obtained. Preferred compositions and preparations according to the present invention are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other

synthetic solvents; antibacterial agents such as benzyl alcohol or methyl paraben; antioxidants such as ascorbic acid or sodium bisulfite; chelating agents such as ethylene diaminetetraacetic acid; buffers such as acetates, citrates or phosphates and agents for the adjustment of tonicity such as sodium chloride or dextrose. The parenteral preparation can be enclosed in ampules, disposable syringes or multiple dose vials made of glass or plastic.

Example 1

a. A stirring mixture of 43.0 g of 2-(2-nitrophenyl)acetophenone, 200 ml of 95% ethyl alcohol, 31.2 g of sodium acetate and 24.3 g of hydroxylamine hydrochloride in 100 ml of water is refluxed for 1 hour and then permitted to stand for 16 hours. Thereafter, the precipitate is collected by suction filtration and the filter cake is sequentially washed with 100 ml of 60% aqueous ethyl alcohol, two 100 ml portions of water, dried and recrystallized from 95% ethyl alcohol to provide nearly colorless crystals, mp 119—121°C, of 2-(2-nitrophenyl)acetophenone oxime. [The 2-(2-nitrophenyl)acetophenone starting material is reported in Chem., Absts., *63*, 16237g (1965)].

b. A stirring solution of 5.0 g of 2-(2-nitrophenyl)acetophenone oxime in 10 ml of potassium hydroxide dried pyridine is treated with 5 ml of acetic anhydride in three equal portions. After total addition, the solution is heated on a steam bath for 45 minutes with the exclusion of moisture and then decanted into 50 ml of ice water. An oil separates which solidifies with continued stirring. The solid is successively collected by vacuum filtration, washed with water, dried under vacuum over potassium hydroxide pellets and recrystallized from 95% ethyl alcohol to provide nearly colorless crystals, mp 63—66°C, of 2-(2-nitrophenyl)-acetophenone oxime acetate.

c. 150 ml of a 1M solution of diborane in tetrahydrofuran are added to a stirring mixture of 9.0 g of 2-(2-nitrophenyl)-acetophenone oxime acetate and 150 ml of tetrahydrofuran over a 20 minute span with ice-water bath cooling. After total addition, the reaction mixture is stirred for 48 hours at ambient temperature before carefully adding, with stirring, 150 ml of a 5% hydrochloric acid solution. After this total addition the tetrahydrofuran is distilled under reduced pressure and the residue is extracted thrice with 200 ml portions of ether. The aqueous phase is basified with 50% sodium hydroxide and the alkaline phase is extracted thrice with 300 ml portions of ether. The combined ether extracts are dried and then concentrated, leaving an oil which in ether, is converted to its hydrochloric acid salt. The salt is collected by vacuum filtration and then washed thoroughly with ether. The dried salt is recrystallized from methyl alcohol to provide colorless crystals, mp 261—263°C., dec., of 2-nitro-$\alpha$-phenylphenethylamine hydrochloride.

d. 19.4 g of 25% (W/W) a sodium methoxide in methyl alcohol solution are added to a suspension of 19.6 g of 2-nitro-$\alpha$-phenylphenethylamine hydrochloride in 100 ml of ethyl alcohol. After the addition, the mixture is stirred with steam bath warming before being filtered. The filter cake is washed with 70 ml of 95% ethyl alcohol and the filtrates are combined and then transferred into a 500 ml Parr hydrogenation bottle which had been charged with 2.0 g of a 10% palladium/carbon catalyst and 20 ml of 95% ethyl alcohol. The mixture is hydrogenated for 3 hours at 50 psig at ambient temperature. Thereafter, the mixture is suction filtered and the filtrate is evaporated, leaving an orange oil which is dissolved in 100 ml of chloroform. The chloroform solution and the washing is extracted twice with 100 ml portions of chloroform. All the chloroform solutions are combined and then sequentially dried, filtered and the filtrate is evaporated, leaving an orange oil which is diluted with 12 ml of cyclohexane. This solution is seeded with a crystalline product obtained from the previously obtained crude oil by crystallization of a sample from a small amount of cyclolhexane, stirred vigorously and permitted to stand at 5°C. for 48 hours. Thereafter, the mother liquor is decanted and the crystalline precipitate is pulverized and collected in a Buchner funnel. The filter cake is washed with cyclohexane, dried and recrystallized from cyclohexane to provide colorless crystals, mp 43—44°C., of 2-amino-phenylphenethylamine.

e. 70 ml of hydrochloric acid saturated absolute ethyl alcohol are added dropwise to a stirred mixture of 7.0 g of 2-amino-$\alpha$-phenylphenethylamine hydrochloride, salt of step d, and 70 ml of triethylorthoformate. After total addition, the reaction mixture is refluxed, in the absence of moisture, for 16 hours and then successively cooled, diluted with ether and suction filtered to provide a crystalline material which is dried under vacuum for 5 hours. The product is recrystallized from a methyl alcohol-ether mixture and the resulting crystals are washed with ether and then dried, providing colorless crystals, mp 185—187°C., of 4,5-dihydro-4-phenyl-3H-1,3-benzodiazepine hydrochloride.

Analysis:

| | | | |
|---|---|---|---|
| Calculated for $C_{15}H_{14}N_2 \cdot HCl$: | 69.62%C; | 5.84%H; | 10.82%N. |
| Found: | 69.71%C; | 5.84%H; | 10.83%N. |

Example 2

76.6 of triethylorthoacetate are added to a refluxing solution of 7.1 g of 2-amino-$\alpha$-phenylphenethylamine (Example 1d) and 91 ml of hydrochloric acid saturated absolute ethyl alcohol. After

this addition, refluxing is continued for 16 hours before the mixture is permitted to cool. The cooled mixture is diluted with 1.5 liter of ether and then refrigerated in the absence of moisture for 72 hours. Thereafter, the solvent is decanted and the precipitate is covered with ether. The precipitate is collected by suction filtration and then dried. The dried product is recrystallized from an ethyl alcohol-ether mixture to provide, after drying under vacuum at 40°C, colorless crystals mp 194—197°C., of 4,5-dihydro-2-methyl-4-phenyl-3H-1,3-benzodiazepine hydrochloride.

Analysis:

| | | | |
|---|---|---|---|
| Calculated for $C_{16}H_{16}N_2 \cdot HCl$: | 70.45%C; | 6.28%H; | 10.26%N. |
| Found: | 70.42%C; | 6.29%H; | 10.11%N. |

Example 3

a. A mixture of 27.6 g of acetic anhydride and 16.7 ml of formic acid is heated at 50—67°C for 15 minutes and then cooled to 10—15°C. A solution of 31.8 g of 2-nitro-$\alpha$-phenylphenethylamine, free base of Example 1c, in benzene is added at such a rate as to maintain the reaction mixture's temperature at 10—15°C. After total addition, the suspension is stirred for 48 hours at 50°C and then stirred for an additional 48 hours at ambient temperature. Thereafter, the material is collected by suction filtration and then rinsed well with ether. The filtrate is evaporated under reduced pressure and the resulting solid is combined with the filter cake. The combined solid is dried under vacuum at 40°C for 2 hours and the dried material is dissolved in methylene chloride and this solution is successively washed with 5% hydrochloric acid solution, washed with water, dried, filtered and evaporated leaving a solid. The solid is recrystallized from ethyl alcohol to provide, after drying under vacuum at 90°C., light yellow crystals, mp 151—153°C., of N-formyl-2-nitro-$\alpha$-phenylphenethylamine.

b. A solution of 96 ml of 1M diborane in tetrahydrofuran and 50 ml of tetrahydrofuran is added dropwise to an ice cold suspension of 13 g of N-formyl-2-nitro-$\alpha$-phenylphenethylamine in 150 of tetrahydrofuran. After the effervescence ceases, 28 ml of 5N hydrochloric acid are added dropwise. Thereafter, the solvent is removed under reduced pressure and the resulting oily mixture is basified with a 50% sodium hydroxide aqueous solution. The basified mixture is extracted with several portions of ether and the combined ether extracts are successively dried, filtered and evaporated, providing an oil. The oil is dissolved in benzene and this solution is treated with ethereal hydrochloric acid. The solvent is removed and the resulting material is recrystallized from isopropyl alcohol to provide, after drying over xylene for 24 hours, light yellow crystals, mp 192—196°C., of N-methyl-2-nitro-$\alpha$-phenylphenethylamine hydrochloride.

c. A solution of 0.5 g of N-methyl-2-nitro-$\alpha$-phenylphenethylamine hydrochloride in 20 ml of ethyl alcohol is added to a Parr hydrogenation bottle previously charged with 0.2 g of platinum dioxide, Adam's catalyst, and 5 ml of ethyl alcohol. This is pressurized to 50 psi with hydrogen for 5 minutes, then to 15 psi and placed on a Parr shaker for 30 minutes. The mixture is filtered through a fine sintered glass funnel and the filtrate is evaporated. The resulting material is recrystallized from isopropyl alcohol to provide, after drying, colorless crystals, mp 190—191°C., of 2-amino-N-methyl-$\alpha$-phenylphenethylamine hydrochloride.

d. 40 ml hydrochloric acid saturated absolute ethyl alcohol are added dropwise to a stirred mixture of 4.0 g of 2-amino-N-methyl-$\alpha$-phenylphenethylamine hydrochloride and 40 ml of triethyl orthoformate. After this addition, the resulting solution is successively refluxed for 16 hours, cooled, diluted with ether and suction filtered, providing a crystalline material. The material is recrystallized by dissolving in methyl alcohol, filtering and diluting with ether to provide, after drying, colorless crystals, mp 251—254°C., of 4,5-dihydro-3-methyl-4-phenyl-3H-1,3-benzodiazepine hydrochloride.

Analysis:

| | | | |
|---|---|---|---|
| Calculated for $C_{16}H_{16}N_2 \cdot HCl$: | 70.45%C; | 6.28%H; | 10.26%N. |
| Found: | 70.44%C; | 6.19%H; | 10.50%N. |

Example 4

40 ml of hydrochloric acid saturated absolute ethyl alcohol are added dropwise to a stirred mixture of 4.0 g of 2-amino-N-methyl-$\alpha$-phenylphenethylamine hydrochloride (Example 3c) and 40 ml of triethyl orthoacetate. After total addition the reaction mixture is successively refluxed for 2 hours, cooled, diluted with ether and filtered to collect a solid material. The material is recrystallized from a methyl alcohol-ether mixture to provide a white, solid material which is dried to provide a crystalline product mp 240—243°C., of 4,5-dihydro-2,3-dimethyl-4-phenyl-3H-1,3-benzodiazepine hydrochloride.

Analysis:

| | | | |
|---|---|---|---|
| Calculated for $C_{17}H_{18}N_2 \cdot HCl$: | 71.19%C; | 6.68%H; | 9.77%N. |
| Found: | 71.43%C; | 6.59%H; | 9.73%N. |

Other compounds of the invention can be prepared from appropriate starting materials by procedures consistent with those in the above examples.

Example 5

*4,5-Dihydro-2-methyl-4-phenyl-3-propyl-3H-1,3-benzodiazepine hydrochloride*

90 ml of Ethanolic hydrogen chloride is added dropwise to a stirred suspension of 9.00 g of 2-amino-$\alpha$-phenyl-N-propylphenethylamine in 90 ml of triethyl orthoacetate. After complete addition, the mixture is refluxed for 2 hours. The ethanol is slowly distilled from the mixture and additional ethanolic hydrogen chloride (50 ml) is added. The mixture is then distilled to a volume of 20—30 ml. 100 ml of ether is added causing precipitation of the hydrochloride salt. The salt is collected, washed with ether (100 ml) and dried to give white crystals, mp 272—275°C of 4,5-dihydro-2-methyl-4-phenyl-3-propyl-3H-1,3-benzodiazepine hydrochloride.

Analysis:

| | | | |
|---|---|---|---|
| Calculated for $C_{19}H_{22}N_2HCl$: | 72.48%C; | 7.36%H; | 8.90%N. |
| Found: | 72.25%C; | 7.34%H; | 8.90%N. |

Example 6

*N-cyclohexylmethyl-2-methyl-4-phenyl-3H-1,3-benzodiazepine hydrochloride*

250 g of 2-Amino-N-cyclohexylmethyl-$\alpha$-phenylphenethylamine dihydrochloride is heated in 2.40 ml of refluxing triethyl orthoacetate and 0.75 ml of acetic acid for 1.5 hours, after which the volatile components are distilled from the reaction mixture. The residual solvents are removed *in vacuo* (aspirator). The resulting solid is dissolved in 25 ml of 5% hydrochloric acid and 10 ml of methylene chloride. The biphasic mixture is poured onto 100 ml of water and the resulting biphasic mixture is extracted with methylene chloride (2×100 ml). The combined methylene chloride extracts are dried over anhydrous magnesium sulfate and the methylene chloride evaporated affording a pale yellow oil. The oil is dissolved in 50 ml of ether. Etherial hydrogen chloride is added precipitating the salt as an oily solid. The ether is evaporated and the oily solid is dissolved in 10 ml of methanol. 50 ml of ethyl acetate is then added. The methanol is boiled from solution, followed by cooling. Hexane is slowly added until a slight cloudiness appears. Scratching produces white crystals, mp 256—258°C, of N-cyclohexylmethyl-2-methyl-4-phenyl-3H-1,3-benzodiazepine hydrochloride.

Analysis:

| | | | |
|---|---|---|---|
| Calculated for $C_{23}H_{28}N_2 \cdot HCl$: | 74.88%C; | 7.92%H; | 7.59%N. |
| Found: | 74.71%C; | 8.02%H; | 7.55%N. |

Example 7

*2-Ethyl-3-methyl-4-phenyl-3H-1,3-benzodiazepine hydrochloride*

6.00 g of 2-Amino-N-methyl-$\alpha$-phenylphenethylamine dihydrochloride is heated in 8.1 ml of refluxing triethyl orthopropionate and 2.4 ml of acetic acid for 2 hours, after which the volatile solvents are distilled from the reaction mixture. The less volatile solvents are then distilled *in vacuo* (aspirator) from the mixture. The resulting oil is dissolved in 20 ml of 5% hydrochloric acid and the acidic solution is poured onto 100 ml of water. The aqueous phase is washed with 100 ml of ether, basified with 50% sodium hydroxide and extracted with dichloromethane (2×200 ml). The combined dichloromethane extracts are dried over anhydrous magnesium sulfate and the dichloromethane evaporated affording a colorless oil. The oil is dissolved in 100 ml of ether. Ethereal hydrochloric acid is added precipitating the salt as an oily solid. The ether is evaporated from the mixture and the resulting oil is recrystallized from 20 ml of acetone providing crystals, mp 253—255°C dec, of 2-ethyl-3-methyl-4-phenyl-3H-1,3-benzodiazepine hydrochloride.

Analysis:

| | | | |
|---|---|---|---|
| Calculated for $C_{18}H_{20}N_2 \cdot HCl$: | 71.87%C; | 7.04%H; | 9.31%N. |
| Found: | 71.69%C; | 6.87%H; | 9.29%N. |

Example 8

*3-Benzyl-4,5-dihydro-2-methyl-4-phenyl-3H-1,3-benzodiazepine hydrochloride*

6.00 g of 2-Amino-N-benzyl-α-phenylphenethylamine dihydrochloride is heated in 17.64 ml of refluxing triethyl orthoacetate and 5.58 ml of acetic acid for 24 hours, after which the solvents are distilled from the reaction flask first at atmospheric pressure followed by distillation *in vacuo* (aspirator). The resulting oil is dissolved in 5% hydrochloric acid:methylene chloride (20 ml: 10 ml) and poured onto 100 ml of water containing 10 ml of 37% hydrochloric acid. The biphasic acidic mixture is washed with ether (2×100 ml), basified with 10 ml of 50% sodium hydroxide and extracted with methylene chloride (2×200 ml). The combined methylene chloride extracts are dried over anhydrous magnesium sulfate and the methylene chloride evaporated. The resulting oil is dissolved in ether (100 ml). Ethereal hydrochloric acid (20 ml) is added precipitating the hydrochloride salt. The precipitate is collected and washed with 60 ml of hot acetone affording white crystals, mp 245—247°C, of 3-benzyl-4,5-dihydro-2-methyl-4-phenyl-3H-1,3-benzodiazepine hydrochloride.

Analysis:

| | | | |
|---|---|---|---|
| Calculated for $C_{23}H_{22}N_2 \cdot HCl$: | 76.03%C; | 6.38%H; | 7.71%N. |
| Found: | 75.59%C; | 6.45%H; | 7.64%N. |

Example 9

*4,5-Dihydro-3-ethyl-2-methyl-4-phenyl-3H-1,3-benzodiazepine hydrochloride*

4.00 g of 2-Amino-N-ethyl-α-phenylphenethylamine dihydrochloride is heated in 14.4 ml of triethyl orthoacetate and 4.44 ml of acetic acid for 24 hours after which the solvents are removed first by distillation at atmospheric pressure, followed by distillation *in vacuo* (aspirator). The resulting oil is dissolved in 20 ml of 5% hydrochloric acid. The acidic mixture is poured onto 200 ml of water containing 20 ml of 37% hydrochloric acid. The bisphasic mixture is washed with 100 ml of ether, basified with 50% sodium hydroxide solution and extracted with methylene chloride (2×200 ml). The combined extracts are dried over anhydrous magnesium sulfate and evaporated affording a yellow oil. The oil is dissolved in 100 ml of ether. 10 ml of Ethereal hydrochloric acid is added precipitating the hydrochloride salt. The salt is collected and washed with hot acetone affording pale yellow crystals, mp 249—252°C, of 4,5-dihydro-3-ethyl-2-methyl-4-phenyl-3H-1,3-benzodiazepine hydrochloride.

Analysis:

| | | | |
|---|---|---|---|
| Calculated for $C_{18}H_{20}N_2 \cdot HCl$: | 71.87%C; | 7.04%H; | 9.31%N. |
| Found: | 71.57%C; | 7.05%H; | 9.20%N. |

Example 10

*4,5-Dihydro-2,3-dimethyl-4-(4-fluorophenyl)-3H-1,3-benzodiazepine hydrochloride*

A mixture of 4 g of 2-amino-N-methyl-α-(4-fluorophenyl)phenethylamine, 5.6 ml of acetic acid and 18.33 ml of triethyl orthoacetate is refluxed for 2 hours. The solvents are evaporated *in vacuo*. The residue is combined with 5% hydrochloric acid and extracted with ether. The aqueous layer is basified and extracted with ether. The dried (anhydrous sodium sulfate) organic phase is filtered, ethereal hydrochloric acid is added dropwise to the filtrate and the product oils out. The solvent is evaporated and the residue recrystallized from ethanol-ether giving crystals, mp 253—255°C, of 4,5-dihydro-2,3-dimethyl-4-(4-fluorophenyl)-3H-1,3-benzodiazepine hydrochloride.

Analysis:

| | | | |
|---|---|---|---|
| Calculated for $C_{17}H_{17}FN_2 \cdot HCl$: | 66.99%C; | 5.95%H; | 9.19%N. |
| Found: | 66.69%C; | 5.87%H; | 9.01%N. |

Example 11

*4,5-Dihydro-2,3-dimethyl-α-(4-methylphenyl)-3H-1,3-benzodiazepine hydrochloride*

A mixture of 3 g of 2-amino-N-methyl-α-(4-methylphenyl)phenethylamine dihydrochloride, 9.32 g of triethyl orthoacetate and 3.42 g of acetic acid is refluxed for 3 1/4 hours. The solvent is evaporated and approximately 10 ml of 5% hydrochloric acid and 100 ml of water are added. The aqueous layer is extracted with ether. The aqueous layer is basified with 10% sodium hydroxide solution and extracted with ether. Ethereal hydrochloric acid is added and the product precipitates the salt. Recrystallization from ethanol gives crystals, mp 285—288°C, of 4,5-dihydro-2,3-dimethyl-α-(4-methylphenyl)-3H-1,3-benzodiazepine hydrochloride.

Analysis:

| | | | |
|---|---|---|---|
| Calculated for $C_{18}H_{20}N_2 \cdot HCl$: | 71.87%C; | 7.04%H; | 9.31%N. |
| Found: | 71.85%C; | 7.11%H; | 9.28%N. |

Example 12

*4,5-Dihydro-2,3-dimethyl-4-(4-methoxyphenyl)-3H-1,3-benzodiazepine*

A stirred solution of 4.10 g of 2-amino-α-(4-methoxyphenyl)-N-methylphenethylamine, 16.22 g of triethyl orthoacetate and 5.6 ml of glacial acetic acid is held under reflux (bath temperature 112°C) for 2 hours. After standing overnight at ambient temperature the solution is concentrated *in vacuo* (pump) at 90°C on a rotary evaporator. An ethereal solution of the residual oil is washed with 5% hydrochloric acid. The aqueous phase is made alkaline with 50% sodium hydroxide solution and the mixture is extracted twice with 25 ml-portions of methylene chloride. The combined, (dried over anhydrous sodium sulfate) organic phase is evaporated to afford a solid. Recrystallization from 10 ml of acetonitrile provides tan crystals, mp 130—132°C of 4,5-dihydro-2,3-dimethyl-4-(4-methoxyphenyl)-3H-1,3-benzodiazepine.

Analysis:

| | | | |
|---|---|---|---|
| Calculated for $C_{18}H_{20}N_2O$: | 77.12%C; | 7.19%H; | 9.99%N. |
| Found: | 77.20%C; | 7.13%H; | 10.05%N. |

Example 13

*4,5-Dihydro-2-ethyl-4-(4-methoxyphenyl)-3-methyl-3H-1,3-benzodiazepine hydrochloride*

A stirred solution of 4.1 g of 2-amino-α-(4-methoxyphenyl)-N-methylphenethylamine, 17.6 g of triethyl orthopropionate and 5.6 ml of glacial acetic acid is heated under reflux for 3 hours with exclusion of moisture. After standing overnight at ambient temperature the solution is concentrated *in vacuo* (pump) at 90°C on a rotary evaporator. A solution of the residual oil and 100 ml of methylene chloride is washed with 10% sodium hydroxide solution. The dried (over anhydrous sodium sulfate) organic phase is filtered and concentrated to an oil which is dissolved in 50 ml of anhydrous ether. Treatment with excess ethereal hydrogen chloride provides a precipitate which is collected, washed with ether and dried *in vacuo* at 40°C. Recrystallization from 40 ml of isopropanol provides colorless crystals, mp 240—242°C dec, of 4,5-dihydro-2-ethyl-4-(4-methoxyphenyl)-3-methyl-3H-1,3-benzodiazepine hydrochloride.

Analysis:

| | | | |
|---|---|---|---|
| Calculated for $C_{19}H_{22}N_2 \cdot HCl$: | 68.98%C; | 7.01%H; | 8.47%N. |
| Found: | 68.66%C; | 6.98%H; | 8.30%N. |

Example 14

*4,5-Dihydro-2-ethyl-4-phenyl-3H-1,3-benzodiazepin hydrochloride*

A stirred solution of 3.33 g of 2-amino-α-phenylphenethylamine, 17.63 g of triethyl orthopropionate and 5.6 ml of glacial acetic acid is heated under reflux with exclusion of moisture for 2 1/2 hours. Volatile components are removed on a rotary evaporator at 90°C under reduced pressure (pump). A solution of the residual oil and 100 ml of methylene chloride is washed with 10% sodium hydroxide solution, dried over anhydrous sodium sulfate, filtered and concentrated to an oil. A solution of the oil and 70 ml of anhydrous ether is treated with excess ethereal hydrogen chloride. The precipitate is collected, washed with anhydrous ether and dried. Recrystallization from 90 ml of isopropanol provides colorless crystals, mp 242—245°C, of 4,5-dihydro-2-ethyl-4-phenyl-3H-1,3-benzodiazepine hydrochloride

Analysis:

| | | | |
|---|---|---|---|
| Calculated for $C_{17}H_{18}N_2 \cdot HCl$: | 71.20%C; | 6.68%H; | 9.77%N. |
| Found: | 71.23%C; | 6.65%H; | 9.70%N. |

Example 15

*4,5-Dihydro-4-(3,4-dimethoxyphenyl)-2,3-dimethyl-3H-1,3-benzodiazepine hydrochloride*

A mixture of 3.5 g of α-(3,4-dimethoxyphenyl)-N-methyl-2-nitrophenethylamine, 11.9 g of triethyl orthoacetate and 4.6 ml of acetic acid is refluxed for 2 1/2 hours. The solvents are evaporated

and the residue partitioned between 5% hydrochloric acid and ether. The aqueous layer is separated, basified, extracted with ether and dried over anhydrous sodium sulfate. Ethereal hydrochloric acid is added to the filtered ether extract and the product oils out. The mixture is evaporated to dryness and the residue recrystallized from ethanol-ether, giving colorless crystals, mp 222—226°C, of 4,5-dihydro-4-(3,4-dimethoxyphenyl)-2,3-dimethyl-3H-1,3-benzodiazepine hydrochloride.

Analysis:

| | | | |
|---|---|---|---|
| Calculated for $C_{19}H_{22}N_2O_2 \cdot HCl$: | 65.89%C; | 6.68%H; | 8.08%N. |
| Found: | 65.84%C; | 6.62%H; | 8.06%N. |

Example 16

*4,5-Dihydro-4-(3,4-dimethoxyphenyl)-2,3-dimethyl-3H-1,3-benzodiazepine hydrochloride*

A mixture of 1.1 g of N-acetyl-$\alpha$-(3,4-dimethoxyphenyl)-N-methyl-2-aminophenethylamine in 40 ml of methylene chloride and 0.26 g of phosphorous oxychloride is refluxed 18 hours. The residue is basified with 10% sodium hydroxide solution and extracted with additional methylene chloride. The extract is dried (over anhydrous sodium sulfate) and evaporated. The residue is dissolved in absolute ethanol and ethereal hydrogen chloride is added. The product crystallizes on additon of the ethereal hydrogen chloride, ether giving colorless crystals, mp 220—226°C, of 4,5-dihydro-4-(3,4-dimethoxyphenyl)-2,3-dimethyl-3H-1,3-benzodiazepine.

Analysis:

| | | | |
|---|---|---|---|
| Calculated for $C_{19}H_{22}N_2O_3 \cdot HCl$: | 65.80%C; | 6.68%H; | 8.08%N. |
| Found: | 65.53%C; | 6.70%H; | 7.87%N. |

Example 17

*4,5-Dihydro-4-(3,4-dimethoxyphenyl)-2-ethyl-3-methyl-3H-1,3-benzodiazepine*

A mixture of 4 g of 2-amino-N-methyl-$\alpha$-(3,4-dimethoxyphenyl)phenethylamine, 14.8 g of triethyl orthopropionate and 5.54 g of acetic acid is refluxed for 2 hours. The solvents are evaporated. The residue is dissolved in methylene chloride, washed with 5% sodium hydroxide solution and water and dried over anhydrous sodium sulfate, filtered and evaporated. The residue is dissolved in ether and ethereal hydrogen chloride is added dropwise. The product which precipitates as the hydrochloride salt is recrystallized from ethanol-ether giving tan crystals, mp 214—216°C, of 4,5-dihydro-4-(3,4-dimethoxyphenyl)-2-ethyl-3-methyl-3H-1,3-benzodiazepine.

Analysis:

| | | | |
|---|---|---|---|
| Calculated for $C_{20}H_{24}N_2O_2 \cdot HCl$: | 66.57%C; | 6.98%H; | 7.76%N. |
| Found: | 66.63%C; | 6.93%H; | 7.81%N. |

Preparation of Intermediates

Example 18

*2-Amino-N-ethyl-$\alpha$-phenylphenethylamine dihydrochloride* (II)

3.00 g of N-Ethyl-2-nitro-$\alpha$-phenylphenethylamine hydrochloride is treated with 100 ml of hot 95% ethanol containing 1.10 g of potassium hydroxide for 15 minutes, after which the mixture is filtered and placed in a Paar flask. 0.15 g of 10% palladium on charcoal is added and the mixture is hydrogenated at room temperature and 50 psia for 3 hours, after which the catalyst is removed. The ethanol is evaporated from the filtrate and the resulting yellow oil is poured onto 100 ml of water. The biphasic aqueous mixture is extracted with methylene chloride (2×100 ml). The combined methylene chloride extracts are dried over anhydrous magnesium sulfate and the methylene chloride evaporated to afford a colorless oil. The oil is dissolved in 50 ml of ether. Ethereal hydrogen chloride (100 ml) is added precipitating the hydrochloride acid salt as an oil. The ethanol is evaporated and the oil is dissolved in 20 ml of i-propanol with heating. Upon cooling, white crystals, mp > 248°C dec, fell from solution providing 2-amino-N-ethyl-$\alpha$-phenylphenethylamine dihydrochloride.

Analysis:

| | | | |
|---|---|---|---|
| Calculated for $C_{16}H_{20}N_2 \cdot 2HCl$: | 61.35%C; | 7.08%H; | 8.94%N. |
| Found: | 61.25%C; | 7.11%H; | 8.95%N. |

Example 19

*2-Amino-α-phenyl-N-propylphenethylamine dihydrochloride* (II)

3.34 g of 2-Nitro-α-phenyl-N-propylphenethylamine hydrochloride is heated in 100 ml of 95% ethanol containing 1.21 g of potassium hydroxide for 15 minutes, after which the precipitated sodium chloride is removed by filtration. The ethanolic filtrate is placed in a Paar flask containing 0.15 g of 10% palladium on charcoal and hydrogenated at 50 psia for 2 hours. The catalyst is then removed and the ethanol evaporated. The resulting yellow oil is poured onto 100 ml of water and the biphasic aqueous mixture is extracted with methylene chloride (2×100 ml). The combined methylene chloride extracts are dried over anhydrous magnesium sulfate and the methylene chloride evaporated affording a yellow oil. The oil is dissolved in 50 ml of ethanol. Ethereal hydrogen chloride (50 ml) is added, precipitating the hydrochloride salt. The ethanol is evaporated and the resulting white powder is recrystallized from 20 ml of i-propanol to give crystals, mp > 243°C dec, of 2-amino-α-phenyl-N-propylphenethylamine dihydrochloride.

Analysis:

| | | | |
|---|---|---|---|
| Calculated for $C_{17}H_{24}N_2Cl_2$: | 62.39%C; | 7.39%H; | 8.56%N. |
| Found: | 62.16%C; | 7.32%H; | 8.49%N. |

Example 20

*2-Amino-N-cyclohexylmethylene-α-phenylphenethylamine dihydrochloride* (II)

7.0 g of N-Cyclohexylmethylene-2-nitro-α-phenylphenethylamine hydrochloride is treated with 200 ml of warm 95% ethanol containing 2.07 of potassium hydroxide for 15 minutes, after which the mixture is filtered and placed in a Paar flask containing 0.35 g of 10% palladium on charcoal. The mixture is hydrogenated at 50 psia for 2 hours. After removal of the catalyst, the ethanol is evaporated and the resulting biphasic mixture is poured onto 200 ml of water. The aqueous mixture is extracted with methylene chloride (2×200 ml). The combined methylene chloride extracts are dried over anhydrous magnesium sulfate and the methylene chloride is evaporated giving a colorless oil. The oil is dissolved in 100 ml of ether. Ethereal hydrogen chloride (200 ml) is added and the salt precipitates from solution. The hydrochloride salt is collected as a gummy powder, which is recrystallized from i-propanol to provide crystals, mp 205°C dec, of 2-amino-N-cyclohexylmethylene-α-phenylphenethylamine dihydrochloride.

Analysis:

| | | | |
|---|---|---|---|
| Calculated for $C_{21}H_{28}N_2{\cdot}2HCl$: | 66.14%C; | 7.93%H; | 7.35%N. |
| Found: | 65.90%C; | 7.86%H; | 7.34%N. |

Example 21

*2-Amino-N-methyl-α-(4-methylphenyl)phenethylamine dihydrochloride* (II)

To a mixture of 10 g of α-(4-methylphenyl)-N-methyl-2-nitrophenethylamine and 150 ml of ethanol, 3.7 g of potassium hydroxide is added. The mixture is stirred for 15 minutes and the potassium chloride filtered off. The solution is hydrogenated at room temperature in the presence of 0.5 g of 10% palladium on charcoal for 2 hours. The catalyst is filtered off and ethereal-hydrogen chloride is added to the ethanolic filtrate. The mixture is evaporated and the residue is recrystallized from ethanol-ether giving crystals, mp 225—228°C of 2-amino-N-methyl-α-(4-methylphenyl)phenethylamine dihydrochloride.

Analysis:

| | | | |
|---|---|---|---|
| Calculated for $C_{16}H_{20}N_2{\cdot}2HCl$: | 61.35%C; | 7.08%H; | 8.91%N. |
| Found: | 61.18%C; | 7.02%H; | 8.87%N. |

Example 22

*2-Amino-α-(4-methoxyphenyl)-N-methylphenethylamine dihydrochloride* (II)

A mixture of 10.02 g of α-(4-methoxyphenyl)-N-methyl-2-nitrophenethylamine hydrochloride, 100 ml of dichloromethane and 100 ml of water is treated with excess 10% sodium hydroxide solution. The dried (over anhydrous sodium sulfate) organic phase is concentrated to afford 8.74 g of a yellow oil. A mixture of the oil, 100 ml of absolute ethanol and 0.5 g of 10% palladium on charcoal catalyst is hydrogenated at 50 psia and ambient temperature on a Parr apparatus. Filtration and concentration of the filtrate on a rotary evaporator affords 10.35 g of an oil. A solution of the oil and 20 ml of methanol is treated with excess ethereal hydrogen chloride. Further dilution with ether affords a gum which

solidifies on trituration with fresh ether. Recrystallization from 25 ml of isopropanol gives crystals, mp 223—226°C dec, of 2-amino-α-(4-methoxyphenyl)-N-methylphenethylamine dihydrochloride.

Analysis:

| | | | |
|---|---|---|---|
| Calculated for $C_{16}H_{20}N_2O{\cdot}2HCl$: | 58.37%C; | 6.74%H; | 8.51%N. |
| Found: | 58.11%C; | 6.93%H; | 8.36%N. |

Example 23

*N-Acetyl-2-amino-α-(3,4-dimethoxyphenyl)-N-methylphenethylamine* (II)

A solution of 3.8 g of N-acetyl-α-(3,4-dimethoxyphenyl)-N-methyl-2-nitrophenethylamine in 100 ml of ethanol is hydrogenated at room temperature and 50 psia over 0.5 g of 10% palladium on charcoal for approximately 20 minutes. The catalyst is filtered and the solvent evaporated giving product. Recrystallization from 95% ethanol gave crystals, mp 139—142°C, of N-acetyl-2-amino-α-(3,4-dimethoxyphenyl)-N-methylphenethylamine.

Analysis:

| | | | |
|---|---|---|---|
| Calculated for $C_{19}H_{24}N_2O_3$: | 69.53%C; | 7.37%H; | 8.53%N. |
| Found: | 69.67%C; | 7.33%H; | 8.56%N. |

Example 24

*1-(4-Fluoro-2-methylphenyl)-2-(2-nitrophenyl)ethanone* (II)

To a mixture of 181 g of o-nitrophenylacetic acid in 400 ml of 1,2-dichloroethane, 78 ml of thionyl chloride is added dropwise at room temperature. The mixture is warmed to approximately 65°C then allowed to stand overnight at ambient temperature. To 400 ml of m-fluorotoluene, 133 g of aluminum chloride is added in portions followed by the dropwise addition of the acid chloride solution keeping the temperature approximately 20—25°C. The mixture is gradually warmed to 60°C and held at 60°C until gas evolution ceases. The reaction mixture is poured into concentrated hydrochloric acid and ice and allowed to stand over the weekend. Additional methylene chloride is added to the reaction mixture. The organic layer is separated, washed with 5% sodium hydroxide solution and water, dried over anhydrous sodium sulfate and filtered. Evaporation of the filtrate gives a black oil. The oil is extracted with boiling isopropyl ether which on cooling yields product. Recrystallization from hexane provides crystals, mp 72—74°C, of 1-(4-fluoro-2-methylphenyl)-2-(2-nitrophenyl)ethanone.

Analysis:

| | | | |
|---|---|---|---|
| Calculated for $C_{15}H_{12}FNO$: | 65.93%C; | 4.43%H; | 5.13%N. |
| Found: | 66.01%C; | 4.47%H; | 5.10%N. |

Example 25

*1-(4-Chlorophenyl)-2-(2-nitrophenyl)ethanone* (II)

A mixture of 46 g of α-(4-chlorobenzoyl)-β-dimethylamino-2-nitrostyrene, 150 ml of dioxane and 50 ml of water is heated under reflux for 18 hours. The solution is concentrated. The residue is extracted with methylene chloride, dried over anhydrous sodium sulfate and evaporated to an oil. The oil is dissolved in a small volume of 95% ethanol and the product crystallizes. Recrystallization from ethanol gives crystals, mp 70—72°C, of 1-(4-chlorophenyl)-2-(2-nitrophenyl)ethanone.

Analysis:

| | | | |
|---|---|---|---|
| Calculated for $C_{14}H_{10}ClNO_3$: | 61.00%C; | 3.66%H; | 5.08%N. |
| Found: | 60.96%C; | 3.68%H; | 5.15%N. |

Example 26

*1-(4-Fluorophenyl)-2-(2-nitrophenyl)ethanone* (III)

To a mixture of 10 g of o-nitrophenylacetic acid in 29 ml of fluorobenzene, 4.3 of thionyl chloride is added dropwise at room temperature. The mixture is warmed at 45°C for 3 1/2 hours. To the cooled solution 8.1 g of aluminum chloride is added in portions. During the addition there is a slight exothermic reaction and the temperature rose to 40°C. The mixture is warmed to 45°C for approximately 1 1/2 hours. The reaction mixture is poured into concentrated hydrochloric acid-ice and extracted with ether. The ether extract is washed with 5% sodium hydroxide solution, water, dried over anhydrous sodium

sulfate and evaporated to give a crude solid. Recrystallization from 95% ethanol gives crystals, mp 80—81°C, of 1-(4-fluorophenyl)-2-(2-nitrophenyl)ethanone.

Analysis:

| | | | |
|---|---|---|---|
| Calculated for $C_{14}H_{10}FNO_3$: | 64.86%C; | 3.89%H; | 5.40%N. |
| Found: | 64.64%C; | 3.94%H; | 5.31%N. |

Example 27

*1-(4-Methoxyphenyl)-2-(2-nitrophenyl)ethanone oxime acetate* (V)

A stirred solution of 15.0 g of 1-(4-methoxyphenyl)-2-(2-nitrophenyl)ethanone oxime and 30 ml of potassium hydroxide dried pyridine is treated dropwise with 15.0 ml of acetic anhydride. After stirring overnight at ambient temperature with exclusion of moisture, the solution is heated for 2 hours on a steam bath. The solution is then decanted into 300 ml of ice water and the resultant biphasic mixture is extracted with a total of 270 ml of methylene chloride. The organic phase is washed once with dilute acetic acid (18 ml of glacial acetic acid diluted with 200 ml of water) and then twice with water. Concentrated of the dried (over anhydrous sodium sulfate) organic phase affords 21.0 g of yellow oil. A solution of the oil and 125 ml of ether is washed sequentially with 50 ml-portions each of 5% hydrochloric acid solution, water, 5% sodium bicarbonate solution and water. The (dried over anhydrous sodium sulfate) organic phase is concentrated to a cloudy yellow oil which is subjected to azeotropic distillation with absolute ethanol. On standing overnight at ambient temperature, small rosettes of crystals form and a rosette is removed for use as seed crystals. The remaining material is dissolved in 40 ml of 95% ethanol, cooled slowly until an oil begins to separate and seeded with the previously isolated crystals. The crystalline precipitate is collected by vacuum filtration, washed with 95% ethanol and dried *in vacuo* to provide crystals, mp 65—68.5°C, of 1-(4-methoxyphenyl)-2-(2-nitrophenyl)ethanone oxime acetate.

Analysis:

| | | |
|---|---|---|
| Calculated for $C_{17}H_{16}N_2O_5$: | 62.19%C; | 4.91%H. |
| Found: | 62.16%C; | 4.85%H. |

Example 28

*1-(4-Methoxyphenyl)-2-(2-nitrophenyl)ethanone oxime* (IV)

A stirred suspension of 46.0 g of 1-(4-methoxyphenyl)-2-(2-nitrophenyl)ethanone and 240 ml of 95% ethanol is treated with a solution of 22.6 g of hydroxylamine hydrochloride, 46.3 g of sodium acetate trihydrate and 130 ml of water. The stirred suspension is heated to reflux and 95% ethanol (180 ml) is added. The mixture is heated fc 3 hours under reflux whereupon a clear yellow-colored solution is obtained. After stirring overnight at ambient temperature, excess ethanol is removed on a rotary evaporator and the residue is diluted with approximately 1 l. of water. An oil separates and crystallizes. The solid is collected, washed with water and dried at 40°C overnight *in vacuo*. Recrystallization from 100 ml of 95% ethanol provides crystals, mp 106—110°C, of 1-(4-methoxyphenyl)-2-(2-nitrophenyl)ethanone oxime.

Analysis:

| | | |
|---|---|---|
| Calculated for $C_{15}H_{14}N_2O_4$: | 62.93%C; | 4.93%H. |
| Found: | 62.95%C; | 4.93%H. |

Example 29

*1-(4-Fluorophenyl)-2-(2-nitrophenyl)ethanone oxime acetate* (V)

To a solution of 72.7 g of 1-(4-fluorophenyl)-2-(2-nitrophenyl)ethanone oxime in 175 ml of pyridine, 70 ml of acetic anhydride is added dropwise. The mixture is warmed on the steam bath for 1 1/2 hours. The reaction mixture is poured into ice water and the solution made slightly acid with hydrochloric acid. The material which precipitates is filtered, dried and recrystallized from 95% ethanol giving crystals, mp 74—75°C, of 1-(4-fluorophenyl)-2-(2-nitrophenyl)ethanone oxime acetate.

Analysis:

| | | | |
|---|---|---|---|
| Calculated for $C_{16}H_{13}FN_2O_4$: | 60.76%C; | 4.14%H; | 8.86%N. |
| Found: | 60.66%C; | 4.13%H; | 8.78%N. |

Example 30

*1-(4-Fluorophenyl)-2-(2-nitrophenyl)ethanone oxime* (IV)

To a solution of 6.62 g of 1-(4-fluorophenyl)-2-(2-nitrophenyl)ethanone in 40 ml of 95% ethanol, 3.55 g of hydroxylamine hydrochloride in 10 ml of water and 4.48 g of sodium acetate in 10 ml of water are added. The mixture is refluxed 3 hours and allowed to stand overnight. The ethanol is removed *in vacuo* and the product is extracted with ether, dried over anhydrous sodium sulfate and evaporated. Recrystallization from 95% ethanol gives crystals, mp 139—142°C, of 1-(4-fluorophenyl)-2-(2-nitrophenyl)ethanone oxime.

Analysis:

| | | | |
|---|---|---|---|
| Calculated for $C_{14}H_{11}FN_2O_3$: | 61.31%C; | 4.04%H; | 10.21%N. |
| Found: | 61.15%C; | 4.08%H; | 10.27%N. |

Example 31

*1-(4-Methylphenyl)-2-(2-nitrophenyl)ethanone oxime* (IV)

To a mixture of 50 g of 1-(4-methylphenyl)-2-(2-nitrophenyl)ethanone in 200 ml of 95% ethanol, a solution of 27.8 g of hydroxylamine hydrochloride in 50 ml of water and a solution of 36.1 g of sodium acetate are added. The reaction mixture is refluxed for 3 hours and allowed to stand overnight at ambient temperature. The product which crystallizes from the reaction mixture is filtered and dried. Recrystallization from 95% ethanol provides crystals, mp 129—132°C, of 1-(4-methylphenyl)-2-(2-nitrophenyl)ethanone oxime.

Analysis:

| | | | |
|---|---|---|---|
| Calculated for $C_{15}H_{14}N_2O_3$: | 66.66%C; | 5.22%H; | 10.36%N. |
| Found: | 66.59%C; | 5.31%H; | 10.45%N. |

Example 32

*1-(4-Methylphenyl)-2-(2-nitrophenyl)ethanone oxime acetate* (V)

To a solution of 47.3 g of 1-(4-methylphenyl)-2-(2-nitrophenyl)ethanone oxime in 100 ml of pyridine, 40 ml of acetic anhydride is added dropwise. The mixture is warmed on the steam bath (internal temperature approximately 80°C) for 1 hour. The mixture is poured into water and the product which crystallizes is filtered giving crystals, mp 95—98°C, of 1-(4-methylphenyl)-2-(2-nitrophenyl)ethanone oxime acetate.

Analysis:

| | | | |
|---|---|---|---|
| Calculated for $C_{17}H_{16}N_2O_4$: | 65.38%C; | 5.20%H; | 8.97%N. |
| Found: | 65.42%C; | 5.16%H; | 9.00%N. |

Example 33

*1-(3,4-Dimethoxyphenyl)-2-(2-nitrophenyl)ethanone oxime* (IV)

A mixture of 154 g of 1-(3,4-dimethoxy)-2-(2-nitrophenyl)ethanone in 600 ml of 95% ethanol, 70.9 g of hydroxylamine hydrochloride in 150 ml of water and 92 g of sodium acetate in 150 ml of water is refluxed 7 hours. The product which crystallizes on standing overnight at room temperature is filtered and dried giving crystals, mp 129—130°C, of 1-(3,4-dimethoxyphenyl)-2-(2-nitrophenyl)ethanone oxime.

Analysis:

| | | | |
|---|---|---|---|
| Calculated for $C_{16}H_{16}N_2H_5$: | 60.75%C; | 5.10%H; | 8.85%N. |
| Found: | 60.75%C; | 5.15%H; | 8.84%N. |

Example 34

*1-(3,4-Dimethoxyphenyl)-2-(2-nitrophenyl)ethanone oxime acetate* (V)

A mixture of 156 g of 1-(3,4-dimethoxyphenyl)-2-(2-nitrophenyl)ethanone oxime and 100 g of acetic anhydride is warmed on a steam bath for 30 minutes. The product crystallizes on standing overnight at room temperature. Trituration with hexane gives crystals, mp 118—120°C, of 1-(3,4-dimethoxyphenyl)-2-(2-nitrophenyl)ethanone oxime acetate.

Analysis:

| | | | |
|---|---|---|---|
| Calculated for $C_{18}H_{18}N_2O_6$: | 60.37%C; | 5.06%H; | 7.82%N. |
| Found: | 60.14%C; | 5.11%H; | 7.78%N. |

Example 35

*1-(2-Fluorophenyl)-2-(2-nitrophenyl)ethanone oxime* (IV)

A mixture of 12.3 g of 2′-fluoro-2-(2-nitrophenyl)acetophenone in 80 ml of 95% ethanol, 6.6 g of hydroxylamine hydrochloride in 20 ml of water and 8.53 g of sodium acetate in 20 ml of water is refluxed overnight. The product crystallizes on cooling. Recrystallization from 95% ethanol gives crystals, mp 105—109°C, of 1-(2-fluorophenyl)-2-(2-nitrophenyl)ethanone oxime.

Analysis:

| | | | |
|---|---|---|---|
| Calculated for $C_{14}H_{11}FN_2O_3$: | 61.31%C; | 4.04%H; | 10.21%N. |
| Found: | 61.19%C; | 4.09%H; | 10.25%N. |

Example 36

*1-(2-Fluorophenyl)-2-(2-nitrophenyl)ethanone oxime acetate* (V)

To a solution of 8 g of 1-(2-fluorophenyl)-2-(2-nitrophenyl)ethanone oxime in 20 ml of pyridine, 10 ml of acetic anhydride is added dropwise. The mixture is warmed (steam bath) for 3 1/2 hours, poured into water and extracted with methylene chloride. The methylene chloride extract is washed with 5% hydrochloric acid, dried over anhydrous sodium sulfate, filtered and evaporated. The residual oil is distilled at 5 mm to give 1-(2-fluorophenyl)-2-(2-nitrophenyl)ethanone oxime acetate, bp 133—135°C.

Analysis:

| | | | |
|---|---|---|---|
| Calculated for $C_{16}H_{13}FN_2O_4$: | 60.76%C; | 4.14%H; | 8.86%N. |
| Found: | 60.83%C; | 4.09%H; | 9.11%N. |

Example 37

*α-(4-methoxyphenyl)-2-nitrophenethylamine hydrochloride* (VI)

A stirred ice water chilled solution of 27.8 g of 1-(4-methoxyphenyl)-2-(2-nitrophenyl)ethanone oxime acetate and 250 ml of tetrahydrofuran is treated over 30 minutes with 360 ml of 0.94 M borane in tetrahydrofuran (four-fold excess of boron hydride). After total addition, the solution is stirred for 1 hour at ice-bath temperature, followed by stirring at ambient temperature for 4 hours. The solution is then allowed to stand for 2 days at ambient temperature. The stirred solution is chilled with an ice water bath and quenched by dropwise addition of 100 ml of 5% hydrochloric acid. A colorless precipitate separates and the mixture is stirred for 2 hours with cooling. Glacial acetic acid (15 ml) is added, followed by stirring for 2 hours and then standing overnight at ambient temperature. The stirred suspension is then treated with 300 ml of 10% sodium hydroxide solution, followed by removal of excess tetrahydrofuran on a rotary evaporator. The residual biphasic mixture is extracted thrice with 200 ml-portions of methylene chloride. The combined organic phase is dried over anhydrous sodium sulfate, vacuum filtered and evaporated to an orange colored oil. A solution of the oil and 200 ml of anhydrous ether is treated with a slight excess of ethereal hydrogen chloride. The precipitate is collected by vacuum filtration. The filter cake is washed twice with ether and dried *in vacuo* at 40°C over sodium hydroxide pellets. Recrystallization of the crude product from 600 ml of 95% ethanol gives 16.1 g of slightly yellow crystals, mp 240—242°C, of α-(4-methoxyphenyl)-2-nitrophenethylamine hydrochloride.

Analysis:

| | | |
|---|---|---|
| Calculated for $C_{15}H_{16}N_2O_3 \cdot HCl$: | 58.35%C; | 5.55%H. |
| Found: | 58.21%C; | 5.25%H. |

Example 38

*α-(4-methylphenyl)-2-nitrophenethylamine hydrochloride* (VI)

To a cooled solution of 48 g of 1-(4-methylphenyl)-2-(2-nitrophenyl)ethanone oxime acetate in 350 ml of tetrahydrofuran in an atmosphere of nitrogen, 594 ml 1.01 M borane in tetrahydrofuran is added dropwise. The internal temperature remains approximately 0—20°C during the addition. The

mixture is stirred for 45 minutes at ice bath temperature and then stirred overnight at ambient temperature. The mixture is cooled and the complex is decomposed by cautious addition of 6N hydrochloric acid. A precipitate gradually forms and the mixture is stirred overnight. Tetrahydrofuran is evaporated *in vacuum* and 10% sodium hydroxide solution is added to the residue. The product is extracted with ether and dried over anhydrous sodium sulfate. Ethereal hydrogen chloride is added to the solution and the product precipitates as the hydrochloric salt. Recrystallization from ethanol gives crystals, mp 265—269°C, of α-(4-methylphenyl)-2-nitrophenethylamine hydrochloride.

Analysis:

| | | | |
|---|---|---|---|
| Calculated for $C_{15}H_{16}N_2O_2 \cdot HCl$: | 61.54%C; | 5.85%H; | 9.57%N. |
| Found: | 61.57%C; | 6.00%H; | 9.44%N. |

Example 39

*α-(3,4-Dimethoxyphenyl)-2-nitrophenethylamine hydrochloride* (VI)

To a cooled mixture of 92.6 g of 1-(3,4-dimethoxyphenyl)-2-(2-nitrophenyl)ethanone oxime acetate in 600 ml of tetrahydrofuran in an atmosphere of nitrogen, a solution of 1019 ml of 0.98 M borane in tetrahydrofuran is added dropwise. The mixture is allowed to stand overnight at ambient temperature. The mixture is cooled and 250 ml of 5% hydrochloric acid is added dropwise. The solvent is evaporated *in vacuo* and the residue treated with 10% sodium hydroxide solution. The free amine is extracted with ether, dried over anhydrous sodium sulfate and filtered. Ethereal hydrogen chloride is added dropwise to the filtrate and the product precipitates as the hydrochloride salt. Recrystallization from methanolether gives crystals, mp 229—231°C, of α-(3,4-dimethoxyphenyl)-2-nitrophenethylamine hydrochloride.

Analysis:

| | | | |
|---|---|---|---|
| Calculated for $C_{16}H_{18}N_2O_4 \cdot HCl$: | 56.73%C; | 5.65%H; | 8.27%N. |
| Found: | 56.63%C; | 5.65%H; | 8.22%N. |

Example 40

*α-(4-Fluorophenyl)-2-nitrophenethylamine hydrochloride* (VI)

In an atmosphere of nitrogen, 897 ml of 0.98 M borane in tetrahydrofuran is added dropwise to a cooled solution of 69.2 g of 1-(4-fluorophenyl)-2-(2-nitrophenyl)ethanone oxime acetate in 500 ml of tetrahydrofuran. The mixture is allowed to stand over the weekend. The mixture is cooled and 200 ml of 5% hydrochloric acid is added dropwise. The tetrahydrofuran is evaporated and the residue treated with 10% sodium hydroxide solution. The free amine is extracted with ether and dried over anhydrous sodium sulfate. The ether solution is treated with ethereal hydrogen chloride and the product is recrystallized from ethanol to give crystals, mp 239—243°C, of α-(4-fluorophenyl)-2-nitrophenethylamine hydrochloride.

Analysis:

| | | | |
|---|---|---|---|
| Calculated for $C_{14}H_{13}FN_2O_2 \cdot HCl$: | 56.67%C; | 4.76%H; | 9.44%N. |
| Found: | 56.86%C; | 4.70%H; | 9.36%N. |

Example 41

*N-Ethyl-2-nitro-α-phenylphenethylamine hydrochloride* (VIII)

A 1 M solution of borane-tetrahydrofuran complex in tetrahydrofuran (42 ml) is added dropwise to a stirred cooled suspension of 6.00 g of N-acetyl-2-nitro-α-phenylphenethylamine in 60 ml of tetrahydrofuran. After complete addition the mixture is allowed to stir at room temperature for 24 hours, after which the excess borane is decomposed by the subsequent additions of 20 ml of 5% hydrochloric acid and 2 ml of acetic acid. The mixture is then basified with 20 ml of 50% sodium hydroxide solution. The tetrahydrofuran is evaporated from the biphasic mixture and the remaining aqueous phase is poured onto 100 ml of water. The aqueous mixture is extracted with methylene chloride (2×100 ml). The combined methylene chloride extracts are dried over anhydrous magnesium sulfate and evaporate to afford a yellow oil which is dissolved in 50 ml of ether. Ethereal hydrogen chloride (100 ml) is added precipitating the salt as an oil. The ether is evaporated and the remaining oil is dissolved in 20 ml of 100% ethanol and allowed to crystallize to provide crystals, mp 216—225°C dec, of N-ethyl-2-nitro-α-phenylphenethylamine hydrochloride.

Analysis:

| | | | |
|---|---|---|---|
| Calculated for $C_{16}H_{18}N_2O_2 \cdot HCl$: | 62.64%C; | 6.24%H; | 9.13%N. |
| Found: | 62.53%C; | 6.20%H; | 9.22%N. |

Example 42

*2-Nitro-α-phenyl-N-propylphenethylamine hydrochloride* (VIII)

1 M borane-tetrahydrofuran complex (42 ml) is added dropwise to a cooled, stirred suspension of 6.00 g of 2-nitro-α-phenyl-N-propionylphenethylamine in 60 ml of tetrahydrofuran. After complete addition, the mixture is allowed to stir at room temperature for 24 hours after which the excess borane is decomposed by the successive additions of 20 ml of 5% hydrochloric acid and 2 ml of acetic acid. The acidic mixture is allowed to stir an additional 30 minutes, after which the mixture is basified with 20 ml of 50% sodium hydroxide solution. The mixture is then poured onto 100 ml of water and the aqueous phase extracted with methylene chloride (2×100 ml). The combined methylene chloride extracts are dried over anhydrous magnesium sulfate and the methylene chloride evaporated affording a yellow oil, which is dissolved in 50 ml of ether. Ethereal hydrogen chloride (100 ml) is added precipitating the hydrochloride salt as an oil. The ether is evaporated and the resulting gum is dissolved in 40 ml of hot isopropanol. Upon cooling white crystals, mp 189—192°C, of 2-nitro-α-phenyl-N-propylphenethylamine hydrochloride falls from solution.

Analysis:

| | | | |
|---|---|---|---|
| Calculated for $C_{17}H_{20}N_2O_2 \cdot HCl$: | 63.65%C; | 6.60%H; | 8.73%N. |
| Found: | 63.64%C; | 6.88%H; | 8.38%N. |

Example 43

*N-Benzyl-2-nitro-α-phenylphenethylamine hydrochloride* (VIII)

1 M Borane-tetrahydrofuran complex (30 ml) is added dropwise to a cooled, stirred solution of 9.00 g of N-benzoyl-2-nitro-α-phenylphenethylamine in 90 ml of tetrahydrofuran. After complete addition the mixture is stirred at room temperature for 4 hours. Additional tetrahydrofuran (100 ml) and 1 M borane-tetrahydrofuran complex (30 ml) are added to aid in the dissolution of the reactants. The mixture is allowed to stir at room temperature for 24 hours after which the excess borane is decomposed by the subsequent addition of 45 ml of 5% hydrochloric acid and 5 ml of acetic acid. The acidic mixture is stirred at room temperature for an additional 0.5 hours after which 25 ml of 50% sodium hydroxide solution is added. The tetrahydrofuran is evaporated from the mixture and the resulting aqueous mixture is poured onto 125 ml of water. The aqueous phase is extracted with methylene chloride (1×150 ml, 1×100 ml). The combined methylene chloride extracts are dried over anhydrous magnesium sulfate and evaporated to give a solid which recrystallizes from 150 ml of 100% ethanol giving flocullant crystals. The ethanolic filtrate is acidified with 100 ml of ethereal hydrogen chloride. Upon prolonged standing the hydrochloride salt falls from solution. Recrystallization of the salt from 40 ml of ethanol gives yellow granules, mp 215—218°C, of N-benzyl-2-nitro-α-phenylphenethylamine hydrochloride.

Analysis:

| | | | |
|---|---|---|---|
| Calculated for $C_{21}H_{20}N_2O_2 \cdot HCl$: | 68.38%C; | 5.74%H; | 7.59%N. |
| Found: | 68.11%C; | 5.58%H; | 7.66%N. |

Example 44

*N-Cyclohexylmethylene-2-nitro-α-phenylphenethylamine hydrochloride* (VIII)

1 M Borane-tetrahydrofuran complex (60 ml) is added dropwise to a stirred, cooled suspension of 9.00 g of N-cyclohexylcarbonyl-2-nitro-α-phenylphenethylamine in 90 ml of tetrahydrofuran. After complete addition the mixture is allowed to stir for 4 hours. Additional borane (30 ml) and tetrahydrofuran (100 ml) is added to complete dissolution of materials. The mixture is stirred an additional 16 hours. After cooling of the mixture, the excess borane is decomposed by the subsequent additions of 45 ml of 5% hydrochloric acid and 4 ml of acetic acid. The mixture is stirred for 1/2 hour before the addition of 25 ml of 50% sodium hydroxide solution. The tetrahydrofuran is evaporated from the mixture and the resulting biphasic mixture is poured onto 125 ml of water. The aqueous mixture is extracted with methylene chloride (1×150 ml, 1×100 ml). The combined methylene chloride extracts are dried over anhydrous magnesium sulfate and the methylene chloride is evaporated giving a yellow oil. The oil is dissolved in 100 ml of ether. Ethereal hydrogen chloride (200 ml) is added. Upon standing, a white precipitate falls from solution. The ether is evaporated and the solid is recrystallized from 50 ml

of ethanol giving crystals, mp 225—228°C, of N-cyclohexylmethylene-2-nitro-α-phenylphenethylamine hydrochloride.

Analysis:

| | | | |
|---|---|---|---|
| Calculated for $C_{21}H_{26}N_2O_2$·HCl: | 67.28%C; | 7.26%H; | 7.47%N. |
| Found: | 67.08%C; | 7.26%H; | 7.51%N. |

Example 45

*α-(4-Methoxyphenyl)-N-methyl-2-nitrophenethylamine hydrochloride* (VIII)

A stirred ice water chilled suspension of 12.3 g of N-formyl-α-(4-methoxyphenyl)-2-nitrophenethylamine and 120 ml of tetrahydrofuran is treated over 20 minutes with 85 ml of 1.01 M borane in tetrahydrofuran. After total addition the solution is stirred for 3 hours with ice water cooling and then allowed to stand 2 days at ambient temperature with exclusion of moisture. The stirred solution is chilled and quenched by dropwise addition of 40 ml of 5% hydrochloric acid and 4 ml of glacial acetic acid. Two hours after total addition the mixture is made alkaline with 50% sodium hydroxide solution, diluted with 50 ml of water and concentrated on a rotary evaporator to remove the tetrahydrofuran. The residual liquid is extracted thrice with 70 ml portions of dichloromethane and the combined organic phase is dried over anhydrous sodium sulfate, filtered and concentrated to an oil. A solution of the oil and 100 ml of ether is treated with ethereal hydrogen chloride giving a gum. The ether-gum mixture is evaporated to a yellow colored amorphous foam which is recrystallized from isopropanol to provide almost colorless crystals, mp 181—185°C, of α-(4-methoxyphenyl)-N-methyl-2-nitrophenethylamine hydrochloride.

Analysis:

| | | | |
|---|---|---|---|
| Calculated for $C_{16}H_{18}N_2O_3$·HCl: | 59.54%C; | 5.93%H; | 8.68%N. |
| Found: | 59.24%C; | 5.81%H; | 8.65%N. |

Example 46

*α-(3,4-Dimethoxyphenyl)-N-methyl-2-nitrophenethylamine hydrochloride* (VIII)

To a cooled mixture of 33.6 g of N-formyl-α-(4-methoxyphenyl)-2-nitrophenethylamine in 500 ml of tetrahydrofuran, 408 ml of 0.98 M of boron hydride/tetrahydrofuran is added dropwise keeping the internal temperature at 0—10°C. The mixture is allowed to stand overnight at ambient temperature. To the cooled mixture 100 ml of 5% hydrochloric acid is added cautiously, followed by addition of 20 ml of glacial acetic acid. The solvent is evaporated *in vacuo* and the residue treated with 10% sodium hydroxide solution. The free base is extracted with ether, dried over anhydrous sodium sulfate and filtered. The product is precipitated as the hydrochloride salt by addition of ethereal hydrogen chloride to the ether extract. Recrystallization from ethanol gives crystals, mp 185—187.5°C, of α-(3,4-dimethoxyphenyl)-N-methyl-2-nitrophenethylamine hydrochloride.

Analysis:

| | | | |
|---|---|---|---|
| Calculated for $C_{17}H_{20}N_2O_4$·HCl: | 57.87%C; | 6.00%H; | 7.94%N. |
| Found: | 57.77%C; | 6.09%H; | 7.93%N. |

Example 47

*α-(4-Fluorophenyl)-N-methyl-2-nitrophenethylamine hydrochloride* (VIII)

To a cooled mixture of 31.1 g of N-formyl-α-(4-fluorophenyl)-2-nitrophenethylamine in 500 ml of tetrahydrofuran, 441 ml of 0.98 M borane in tetrahydrofuran is added dropwise. The mixture is allowed to stand over the weekend. The mixture is cooled and 100 ml of 5% hydrochloric acid and 20 ml of glacial acetic acid are added cautiously. The solvent is evaporated *in vacuum* and the residue treated with 10% sodium hydroxide solution. The free amine is extracted with ether and dried over anhydrous sodium sulfate and filtered. Ethereal hydrogen chloride is added dropwise to the filtrate and the product precipitates as the hydrochloride salt. Recrystallization from ethanol gives crystals, mp 241—243°C, of α-(4-fluorophenyl)-N-methyl-2-nitrophenethylamine hydrochloride.

Analysis:

| | | | |
|---|---|---|---|
| Calculated for $C_{15}H_{15}FN_2O_2$·HCl: | 57.98%C; | 4.87%H; | 9.01%N. |
| Found: | 57.89%C; | 5.15%H; | 9.01%N. |

Example 48

*α-(4-Methylphenyl)-N-methyl-2-nitrophenethylamine hydrochloride* (VIII)

To a cooled solution of 17 g of N-formyl-α-(4-methylphenyl)-2-nitrophenethylamine in 200 ml of tetrahydrofuran, 122 ml of 0.98 M borane in tetrahydrofuran is added dropwise in an atmosphere of nitrogen. The temperature did not exceed 10°C during the addition. The mixture is allowed to stir at room temperature for 4 hours. Excess borane is decomposed by addition of 50 ml of 5% hydrochloric acid and 10 ml of acetic acid. The mixture is allowed to stand over the weekend. Tetrahydrofuran is evaporated *in vacuo*. The residue is treated with 10% sodium hydroxide solution, extracted with ether and the organic phase is dried over anhydrous sodium sulfate and filtered. Ethereal hydrogen chloride is added to the filtrate and the hydrochloride salt precipitates. Recrystallization from ethanol yields crystals, mp 235—238°C, of α-(4-methylphenyl)-N-methyl-2-nitrophenethylamine hydrochloride.

Analysis:

| | | | |
|---|---|---|---|
| Calculated for $C_{16}H_{18}N_2O_2 \cdot HCl$: | 62.64%C; | 6.24%H; | 9.13%N. |
| Found: | 62.50%C; | 6.23%H; | 9.14%N. |

Example 49

*N-Acetyl-2-nitro-α-phenylphenethylamine* (VII)

10.0 g of N-Methyl-2-nitro-α-phenylphenethylamine in 75 ml of toluene is added in a dropwise manner to a cooled, stirred solution of 10.2 ml of acetic anhydride in 100 ml of toluene. Upon completion of addition, the mixture is allowed to remain at room temperature for 72 hours, after which the precipitate is collected, washed with ether (2×50 ml) and dried. Recrystallization from 100% ethanol gives crystals, mp 171—172°C, of N-acetyl-2-nitro-α-phenylphenethylamine.

Analysis:

| | | | |
|---|---|---|---|
| Calculated for $C_{16}H_{16}N_2O_3$: | 67.59%C; | 5.67%H; | 9.85%N. |
| Found: | 67.50%C; | 5.61%H; | 9.95%N. |

Example 50

*N-Propionyl-2-nitro-α-phenylphenethylamine* (VII)

10.0 g of N-methyl-2-nitro-α-phenylphenethylamine in 75 ml of toluene is added in a dropwise manner to a cooled, stirred solution of 10.5 ml of propionic anhydride in 100 ml of toluene. Upon completion of addition, the mixture is allowed to remain at room temperature for 75 hours, after which the precipitate is collected, washed with ether (2×50 ml) and dried to give white powder. Recrystallization from 100% ethanol÷water 70 ml÷30 ml) gives pale yellow needles, mp 149—151°C, of N-propionyl-2-nitro-α-phenylphenethylamine.

Analysis:

| | | | |
|---|---|---|---|
| Calculated for $C_{17}H_{18}N_2O_3$: | 68.44%C; | 6.08%H; | 9.39%N. |
| Found: | 68.30%C; | 5.94%H; | 9.26%N. |

Example 51

*N-Benzoyl-2-nitro-α-phenylphenethylamine* (VII)

10.0 g of 2-nitro-α-phenylphenethylamine in 75 ml of toluene is added dropwise to a stirred, cooled solution of 24.3 g of benzoic anhydride in 75 ml of toluene. After complete addition, the mixture is stirred at room temperature for 2 hours after which the precipitate is collected, washed with 300 ml of ether and partially dried. The precipitate is heated in boiling 400 ml ethanol, cooled and collected giving white needles, mp 185—189°C, of N-benzoyl-2-nitro-α-phenylphenethylamine.

Analysis:

| | | | |
|---|---|---|---|
| Calculated for $C_{21}H_{18}N_2O_2$: | 72.82%C; | 5.24%H; | 8.09%N. |
| Found: | 72.54%C; | 5.29%H; | 8.07%N. |

Example 52

*N-Cyclohexanecarbonyl-2-nitro-α-phenylphenethylamine* (VII)

10.0 g of 2-nitro-α-phenylphenethylamine in 75 ml of toluene is added dropwise to a stirred, cooled solution of 14.4 ml of cyclohexylcarbonyl chloride in 75 ml of toluene and 25 ml of pyridine.

After complete addition the mixture is stirred at room temperature for 2 hours. The mixture is allowed to stand overnight, after which the tan precipitate is collected and washed with 100 ml of ether. The precipitate is recrystallized from 350 ml of methanol to give yellow needles, mp 210—212°C, of N-cyclohexanecarbonyl-2-nitro-α-phenylphenethylamine.

Analysis:

| | | | |
|---|---|---|---|
| Calculated for $C_{21}H_{24}N_2O_3$: | 71.57%C; | 6.86%H; | 7.95%N. |
| Found: | 71.36%C; | 6.92%H; | 7.99%N. |

Example 53

*N-Formyl-α-(4-methoxyphenyl)-2-nitrophenethylamine* (VII)

A suspension of 14.0 g of α-(4-methoxyphenyl)-2-nitrophenethylamine hydrochloride, and 250 ml of water is treated with 70 ml of 10% sodium hydroxide solution. The mixture is twice extracted with 200 ml-portions of methylene chloride and the combined organic phase is dried over anhydrous sodium sulfate. Vacuum filtration and concentration affords 12.7 g of a yellow-colored oil. A solution of the oil and 200 ml of methyl formate is heated at 80—83°C (bath temperature) for 3 days in a 300 ml Parr stainless steel bomb. After cooling to room temperature the bomb is opened and the crystalline precipitate is collected by vacuum filtration. The filter cake is washed once with methyl formate and dried *in vacuo* at 40°C to give slightly yellow-colored crystals, mp 151—153°C, of N-formyl-α-(4-methoxyphenyl)-2-nitrophenethylamine.

Analysis:

| | | | |
|---|---|---|---|
| Calculated for $C_{16}H_{16}N_2O_4$: | 63.99%C; | 5.37%H; | 9.33%N. |
| Found: | 63.92%C; | 5.29%H; | 9.37%N. |

Example 54

*N-Formyl-α-(4-methylphenyl)-2-nitrophenethylamine* (VII)

A mixture of 19.6 g of α-(4-methylphenyl)-2-nitrophenethylamine and 230 ml of methyl formate is placed in a Parr bomb at 80°C overnight. The reaction is cooled and the solvent removed *in vacuo* giving an off-white solid. The product is recrystallized from 95% ethanol giving crystals, mp 130—132°C, of N-formyl-α-(4-methylphenyl)-2-nitrophenethylamine.

Analysis:

| | | | |
|---|---|---|---|
| Calculated for $C_{16}H_{16}N_2O_3$: | 67.59%C; | 5.67%H; | 9.85%N. |
| Found: | 67.58%C; | 5.63%H; | 9.94%N. |

Example 55

*N-Formyl-α-(4-fluorophenyl)-2-nitrophenethylamine* (VII)

A mixture of 36.6 g of α-(4-fluorophenyl)-2-nitrophenethylamine and 230 ml of methylformate in a Parr bomb is placed in an oil bath at 80°C and left over the weekend. On cooling a crystalline precipitate separates giving 34.6 g of product. An analytical sample is recrystallized from 95% ethanol providing crystals, mp 142—145°C, of N-formyl-α-(4-fluorophenyl)-2-nitrophenethylamine.

Analysis:

| | | | |
|---|---|---|---|
| Calculated for $C_{15}H_{13}FN_2O_3$: | 62.50%C; | 4.54%H; | 9.72%N. |
| Found: | 62.62%C; | 4.51%H; | 9.77%N. |

Example 56

*N-Formyl-α-(3,4-dimethoxyphenyl)-2-nitrophenethylamine* (VII)

A mixture of 41.7 g of α-(3,4-dimethoxyphenyl)-2-nitrophenethylamine and 250 ml of methyl formate is placed in a Parr bomb at 80°C and left overnight. The solvent is evaporated and the residue recrystallized from 95% ethanol giving crystals, mp 135—139°C, of N-formyl-α-(3,4-dimethoxyphenyl)-2-nitrophenethylamine.

Analysis:

| | | | |
|---|---|---|---|
| Calculated for $C_{17}H_{18}N_2O_5$: | 61.81%C; | 5.49%H; | 8.48%N. |
| Found: | 61.67%C; | 5.27%H; | 8.42%N. |

Example 57

*N-Acetyl-α-(3,4-dimethoxyphenyl)-N-methyl-2-nitrophenethylamine* (VII)

To a cooled solution of 4.17 g of acetic anhydride in 50 ml of toluene, a solution of 4.31 g of α-(3,4-dimethoxyphenyl)-N-methyl-2-nitrophenethylamine is added dropwise. The mixture is warmed to room temperature and stirred for 2 hours. The solvents are evaporated *in vacuo* to give crystals, mp 113—115°C, of N-acetyl-α-(3,4-dimethoxyphenyl)-N-methyl-2-nitrophenethylamine.

Analysis:

| | | | |
|---|---|---|---|
| Calculated for $C_{19}H_{22}N_2O_5$: | 63.67%C; | 6.18%H; | 7.82%N. |
| Found: | 63.68%C; | 6.20%H; | 7.76%N. |

**Claims for the Contracting States: BE CH DE FR GB IT NL SE**

1. A compound of the formula

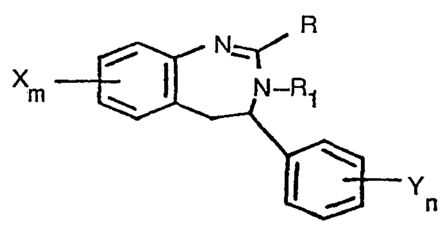

in which R and $R_1$ are the same or different and each can be hydrogen, alkyl of from 1 to 3 carbon atoms, cycloalkyl-$C_1$—$C_3$-alkyl, wherein the cycloalkyl radical has 3—6 carbon atoms, or aralkyl having up to 8 carbon atoms, X and Y are the same or different and each can be chlorine, bromine, fluorine, alkoxy or alkyl each of from 1 to 3 carbon atoms, hydroxy, or trifluoromethyl; m is the integer 0, 1 or 2; n is the integer 0, 1, 2 or 3; the optical antipodes thereof; and a physiologically acceptable salt thereof.

2. A compound as defined in claim 1 in which R and $R_1$ are the same or different and are hydrogen or methyl.

3. A compound as defined in claim 2 in which m and n are each zero.

4. The compound defined in claim 1 which is 4,5-dihydro-2-methyl-4-phenyl-3H-1,3-benzodiazepine or a physiologically acceptable salt thereof.

5. The compound defined in claim 1 which is 4,5-dihydro-4-phenyl-3H-1,3-benzodiazepine.

6. The compound defined in claim 1 which is 4,5-dihydro-3-methyl-4-phenyl-3H-1,3-benzodiazepine.

7. The compound defined in claim 1 which is 4,5-dihydro-2,3-dimethyl-4-phenyl-3H-1,3-benzodiazepine.

8. A compound as claimed in any of claims 1—7, for use in treating depression in mammals including man.

9. A compound as claimed in any of claims 1—7, for use in alleviating pain in mammals including man.

10. A compound as claimed in any of claims 1—7, for use in treating convulsions in mammals including man.

11. A pharmaceutical composition which comprises a physiologically effective amount of a compound defined in claim 1 and a pharmaceutically acceptable carrier therefor.

12. A method for preparing a compound of claim 1 which comprises cyclizing a compound of the formula II

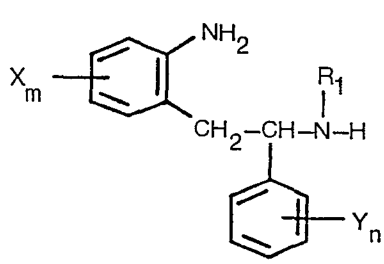

II

wherein $R_1$, X, Y, m and n are as defined in claim 1 with a compound of the formula

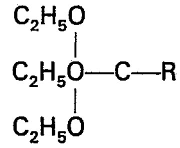

wherein R is as defined in claim 1 in the presence of an acid catalyst and at from 25°C to the reflux temperature of the reaction mixture.

13. The method defined in claim 12, in which the acid catalyst is ethanolic hydrochloric acid.

14. The method defined in claim 12, in which the reaction is carried out under reflux conditions.

**Claims for the Contracting State: AT**

1. A method for preparing a compound of the formula

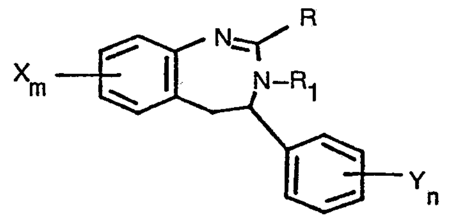

in which R and $R_1$ are the same or different and each can be hydrogen, alkyl of from 1 to 3 carbon atoms, cycloalkyl-$C_1$—$C_3$-alkyl, wherein the cycloalkyl radical has 3—6 carbon atoms, or aralkyl having up to 8 carbon atoms, X and Y are the same or different and each can be chlorine, bromine, fluorine, alkoxy or alkyl each of from 1 to 3 carbon atoms, hydroxy, or trifluoromethyl; m is the integer 0, 1 or 2; n is the integer 0, 1, 2 or 3;
which comprises cyclizing a compound of the formula II

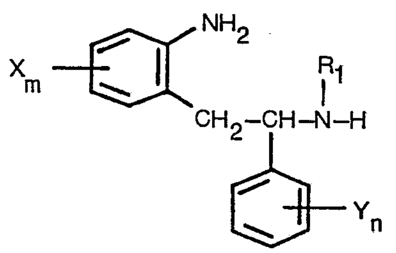

II

wherein $R_1$, X Y m and n are as defined above with a compound of the formula

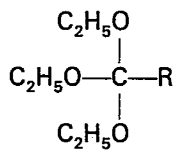

wherein R is as defined above in the presence of an acid catalyst and at from 25°C to the reflux temperature of the reaction mixture.

2. The method defined in claim 1 in which the acid catalyst is ethanolic hydrochloric acid.

3. The method defined in claim 2 in which the reaction is carried out under reflux conditions.

**Revendications pour les Etats contractants: BE CH DE FR GB IT NL SE**

1. Un composé répondant à la formule

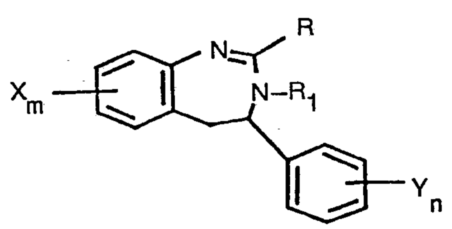

dans laquelle R et $R_1$ sont identiques ou différents et peuvent représenter chacun l'hydrogène, un groupe alcoyle ayant de 1 à 3 atomes de carbone, cycloalcoyl -alcoyle en $C_1$—$C_3$ où le radical cycloalcoyle a de 3 à 6 atomes de carbone, ou aralcoyle ayant jusqu'à 8 atomes de carbone, X et Y sont identiques ou différents et peuvent représenter chacun le chlore, le brome, le fluor, un groupe alcoxy ou alcoyle ayant chacun de 1 à 3 atomes de carbone, hydroxy, ou trifluorométhyle; m est égal à 0, 1 ou 2; n est égal à 0,1, 2 ou 3; ainsi que ses antipodes optiques; et un sel physiologiquement acceptable de ce composé.

2. Un composé selon la revendication 1, caractérisé en ce que R et $R_1$ sont identiques ou différents et sont l'hydrogène ou un groupe méthyle.

3. Un composé selon la revendication 2, caractérisé en ce que m et n sont égaux chacun à zéro.

4. Un composé selon la revendication 1, caractérisé en ce qu'il est la 4,5-dihydro-2-méthyl-4-phényl-3H-1,3-benzodiazépine ou un de ses sels physiologiquement acceptables.

5. Un composé selon la revendication 1, caractérisé en ce qu'il est la 4,5-dihydro-4-phényl-3H-1,3-benzodiazépine.

6. Un composé selon la revendication 1, caractérisé en ce qu'il est la 4,5-dihydro-3-méthyl-4-phényl-3H-1,3-benzodiazépine.

7. Un composé selon la revendication 1, caractérisé en ce qu'il est la 4,5-dihydro-2,3-diméthyl-4-phényl-3H-1,3-benzodiazépine.

8. Un composé selon l'une quelconque des revendications 1 à 7, destiné à être utilisé dans le traitement des dépressions chez les mammifères y compris l'homme.

9. Un composé selon l'une quelconque des revendications 1 à 7, destiné à être utilisé dans le soulagement de la douleur chez les mammifères y compris l'homme.

10. Un composé selon l'une quelconque des revendications 1 à 7, destiné à être utilisé dans le traitement des convulsions chez les mammifères y compris l'homme.

11. Une composition pharmaceutique caractérisée en ce qu'elle contient une quantité physiologiquement efficace d'un composé selon la revendication 1 et un véhicule pharmaceutiquement acceptable.

12. Un procédé pour la préparation d'un composé selon la revendication 1, qui consiste à cycliser un composé de formule II

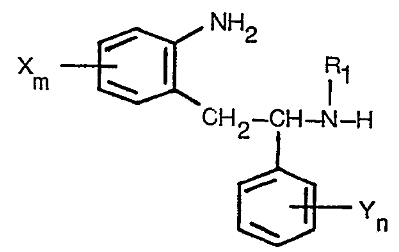

II

dans laquelle $R_1$, X, Y, m et n sont tels que définis dans la revendication 1, avec un composé de formule

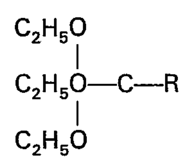

dans laquelle R est tel que défini dans la revendication 1, en présence d'un catalyseur acide et à une température comprise entre 25°C et la température de reflux du mélange réactionnel.

13. Un procédé selon la revendication 12, caractérisé en ce que le catalyseur acide est l'acide chlorhydrique éthanolique.

14. Un procédé selon la revendication 12, caractérisé en ce que la réaction est effectuée dans des conditions de reflux.

**Revendications pour l'Etat contractant: AT**

1. Un procédé pour la préparation d'un composé de formule

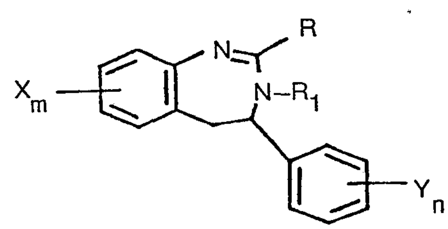

dans laquelle R et $R_1$ sont indentiques ou différents et peuvent être chacun l'hydrogène, un groupe alcoyle ayant de 1 à 3 atomes de carbone, cycloalcoyl-alcoyle en $C_1$—$C_3$ où le radical cycloalcoyle a de 3 à 6 atomes de carbone, ou aralcoyle ayant jusqu'à 8 atomes de carbone, X et Y sont identiques ou différents et peuvent être chacun le chlore, le brome, le fluor, un groupe alcoxy ou alcoyle ayant chacun de 1 à 3 atomes de carbone, hydroxy ou trifluorométhyle; m est égal à 0,1 ou 2; n est égal à 0, 1, 2 ou 3;

lequel procédé consiste à cycliser un composé de formule II

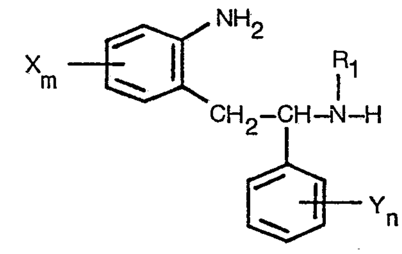

II

dans laquelle $R_1$, X, Y, m et n sont tels que définis ci-dessus, avec un composé de formule

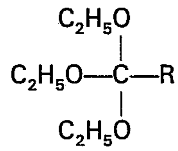

dans laquelle R est tel que défini ci-dessus, en présence d'un catalyseur acide et à une température comprise entre 25°C et la température de reflux du mélange réactionnel.

2. Un procédé selon la revendication 1, caractérisé en ce que le catalyseur acide est l'acide chlorhydrique éthanolique.

3. Un procédé selon la revendication 2, caractérisé en ce que la réaction est effectuée dans des conditions de reflux.

**Patentansprüche für die Vertragsstaaten: BE CH DE FR GB IT NL SE**

1. Eine Verbindung der Formel

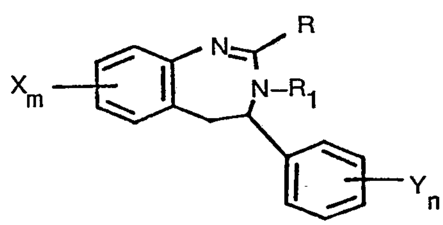

worin R und $R_1$ gleich oder verschieden sind und jeweils Wasserstoff, Alkyl mit 1 bis 3 Kohlenstoffatomen, Cycloalkyl-$C_1$—$C_3$-alkyl, dessen Cycloalkylrest 3—6 Kohlenstoffatome hat, oder Aralkyl mit bis zu 8 Kohlenstoffatomen bedeuten, X und Y gleich oder verschieden sind und jeweils Chlor, Brom, Fluor, Alkoxy oder Alkyl mit jeweils 1 bis 3 Kohlenstoffatomen, Hydroxy oder Trifluormethyl; m die ganze Zahl 0, 1 oder 2 und n die ganze Zahl 0, 1, 2 oder 3 bedeuten, deren optische Antipoden und deren physiologisch verträglichen Salze.

2. Eine Verbindung gemäß Anspruch 1, in der R und $R_1$ gleich oder verschieden sind und Wasserstoff oder Methyl bedeuten.

3. Eine Verbindung gemäß Anspruch 2, in der m und n jeweils 0 bedeuten.

4. Eine Verbindung gemäß Anspruch 1, gekennzeichnet dadurch, daß sie 4,5-Dihydro-2-methyl-4-phenyl-3H-1,3-benzodiazepin oder sein physiologisch verträgliches Salz darstellt.

5. Eine Verbindung gemäß Anspruch 1, gekennzeichnet dadurch, daß sie 4,5-Dihydro-4-phenyl-3H-1,3-benzodiazepin darstellt.

6. Eine Verbindung gemäß Anspruch 1, gekennzeichnet dadurch, daß sie 4,5-Dihydro-3-methyl-4-phenyl-3H-1,3-benzodiazepin darstellt.

7. Eine Verbindung gemäß Anspruch 1, gekennzeichnet dadurch, daß sie 4,5-Dihydro-2,3-dimethyl-4-phenyl-3H-1,3-benzodiazepin darstellt.

8. Eine Verbindung gemäß irgendeinem der Ansprüche 1—7, gekennzeichnet dadurch, daß sie zur Behandlung von Depressionen bei Säugetieren und Menschen verwendet wird.

9. Eine Verbindung gemäß irgendeinem der Ansprüche 1—7, gekennzeichnet dadurch, daß sie zur Schmerzlinderung bei Säugetieren und Menschen verwendet wird.

10. Eine Verbindung gemäß irgendeinem der Ansprüche 1—7, gekennzeichnet dadurch, daß sie für die Behandlung von Krämpfen bei Säugetieren und Menschen verwendet wird.

11. Pharmazeutisches Mittel gekennzeichnet durch einen physiologisch wirksamen Gehalt einer Verbindung gemäß Anspruch 1 und einem pharmazeutisch verträglichen Träger.

12. Verfahren zur Herstellung einer Verbindung gemäß Anspruch 1, gekennzeichnet dadurch, daß man eine Verbindung der Formel II

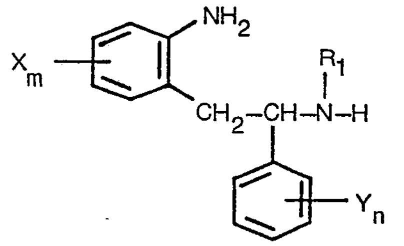

II

in der $R_1$, X, Y, m und n die in Anspruch 1 angegebenen Bedeutungen haben, mit einer Verbindung der Formel

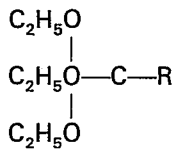

in der R die in Anspruch 1 angegebenen Bedeutungen hat, in Gegenwart eines sauren Katalysators und bei Temperaturen von 25°C bis zur Rückflußtemperatur der Reaktionsmischung cyclisiert.

13. Verfahren gemäß Anspruch 12, gekennzeichnet dadurch, daß der saure Katalysator äthanolische Salzsäure ist.

14. Verfahren gemäß Anspruch 12, gekennzeichnet dadurch, daß die Reaktion unter Rücklußbedingungen durchgeführt wird.

**Patentansprüche für den Vertragsstaat: AT**

1. Verfahren zur Herstellung einer Verbindung der Formel

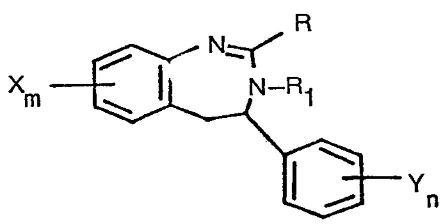

worin R und $R_1$ gleich oder verschieden sind und jeweils Wasserstoff, Alkyl mit 1 bis 3 Kohlenstoffatomen, Cycloalkyl-$C_1$—$C_3$-alkyl, dessen Cycloalkylrest 3—6 Kohlenstoffatome hat, oder Aralkyl mit bis zu 8 Kohlenstoffatomen bedeuten, X und Y gleich oder verschieden sind und jeweils Chlor, Brom, Fluor, Alkoxy oder Alkyl mit jeweils 1 bis 3 Kohlenstoffatomen, Hydroxy oder Trifluormethyl; m die ganze Zahl 0, 1 oder 2 und n die ganze Zahl 0, 1, 2 oder 3 bedeuten, gekennzeichnet dadurch, daß man eine Verbindung der Formel II

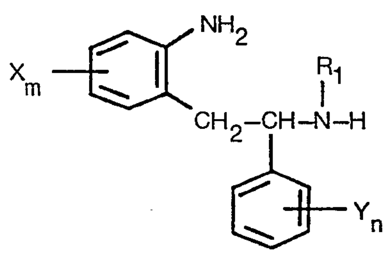

II

in der $R_1$, X, Y, m und n die oben angegebenen Bedeutungen haben, mit einer Verbindung der Formel

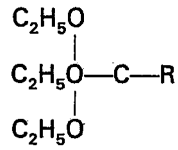

in der R die oben angegebenen Bedeutungen hat, in Gegenwart eines sauren Katalysators und bei Temperaturen von 25°C bis zur Rückflußtemperatur der Reaktionsmischung cyclisiert.

2. Verfahren gemäß Anspruch 1, gekennzeichnet dadurch, daß der saure Katalysator äthanolische Salzsäure ist.

3. Verfahren gemäß Anspruch 2, gekennzeichnet dadurch, daß die Reaktion unter Rückflüßbedingungen durchgeführt wird.